(19) Europäisches Patentamt European Patent Office Office européen des brevets

(11) **EP 4 286 534 A1**

(12) **EUROPEAN PATENT APPLICATION**

(43) Date of publication:
**06.12.2023 Bulletin 2023/49**

(21) Application number: **22176585.2**

(22) Date of filing: **31.05.2022**

(51) International Patent Classification (IPC):
**C12Q 1/6876** (2018.01)

(52) Cooperative Patent Classification (CPC):
**C12Q 1/6876;** C12Q 2600/124; C12Q 2600/156

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Plusvital Ltd.**
**Dun Laoghaire, County Dublin (IE)**

(72) Inventor: **The designation of the inventor has not yet been filed**

(74) Representative: **FRKelly**
**27 Clyde Road**
**Dublin D04 F838 (IE)**

Remarks:
The complete document including Reference Table(s) and the Sequence Listing(s) can be downloaded from the EPO website

(54) **A METHOD OF DETERMINING THE LIKELIHOOD OF A HORSE TO RACE**

(57) The present invention relates to methods for determining or predicting the likelihood of a horse to race or for identifying a horse that is likely to race. The methods are also useful in determining or predicting the athletic or physical performance or ability of a horse, or determining a trait associated with athletic or physical performance or ability of a horse. In an embodiment, the present invention provides a method of determining the likelihood of a horse to race by identifying at least one biomarker in a sample from the horse and comparing the at least one biomarker with a respective reference biomarker.

Figure 4

## Description

### Field of the invention

The present invention relates to methods for determining or predicting the likelihood of a horse to race or for identifying a horse that is likely to race. The methods are also useful in determining or predicting the athletic or physical performance or ability of a horse, or determining a trait associated with athletic or physical performance or ability of a horse.

### Background to the invention

Genetic diversity is critical to maintaining healthy animal populations. For the thoroughbred horse, the relatively small number of founder animals coupled with selective breeding for racing performance traits has led to reduced variation in the gene pool, or high levels of inbreeding when compared to other horse breeds. Inbreeding resulting from genetic improvement and fixing of desirable traits is a feature of livestock and pedigree dog breeds. Inbreeding in the thoroughbred is increasing, which may be driven in part by a popular sire-lines effect. Also, purposeful inbreeding, the mating of close relatives, is common in thoroughbred breeding, with breeders attempting to increase the occurrence of genetic variants for beneficial racing traits in progeny.

Thoroughbred horses are valuable domestic animals bred for competitive racing. The small number of founders and human-mediated selection that disproportionately favours the preservation of popular sire-lines has led to an inbred population with a small effective population size. Purposeful inbreeding is common, with breeders attempting to leverage beneficial genetic variants by choosing mates with shared ancestors such that multiple ancestors may be duplicated in a five-generation pedigree.

To some degree, inbreeding is unavoidable in closed breeding populations. Inbreeding is increasing in the thoroughbred population, and inbreeding can have negative consequences, leading to inbreeding depression, the reduced viability of an individual due to the expression of recessive deleterious mutations. Understanding how inbreeding may affect individual and population fitness is critical to improve breeding management and, for the thoroughbred, may have both economic and welfare implications. For thoroughbred breeders, the breeding goal is to produce viable foals that will have productive racing careers and establish their value by winning races. The number of races that an individual horse participates in and a horse's career duration are considered key indicators of animal health. At a population level, it has been proposed that the success of the thoroughbred industry may be measured by the proportion of horses born that commence racing. Evaluating the negative effect of inbreeding on traits, inbreeding depression, can be achieved by measuring inbreeding and assessing the relationship between inbreeding levels in individual animals and traits that may be impacted by inbreeding. In livestock species, inbreeding negatively impacts on fertility and production traits. Historically inbreeding has been assessed using pedigrees, but this generally underestimates the actual extent of inbreeding. Now, individual inbreeding in an animal can be evaluated by analysing common deoxyribonucleic acid (DNA) segments inherited from the sire and dam using genome-wide single nucleotide polymorphism (SNP) genetic profiles. For example, SNP arrays are widely used in animal species to assess inbreeding and understand the impact of inbreeding on health, production, and welfare traits.

A way to assess inbreeding is to measure runs of homozygosity (ROH) in individual animals. ROH are long stretches of the genome identically inherited from both parents that can occur as the result of shared common ancestors that can occur as the result of active inbreeding or other demographic processes that reduce effective population size. The proportion of the genome covered by ROH affects production traits in livestock populations. Generally, shorter ROH reflect distant inbreeding resulting from a common ancestor many generations back in the pedigree, whereas long ROH reflect a more recent common ancestor. The presence of historic inbreeding may reflect the preservation of favorable genetic variants over time due to selection for genes contributing to success. In humans, deleterious mutations are more common in ROH, particularly long ROH, than in the rest of the genome, while in cattle deleterious mutations are more common in short and medium ROH regions, which may be due to linkage with selected gene variants.

In thoroughbreds, a lower-than-expected mutational load has led to the hypothesis that existing selection processes are effective in purging deleterious mutations. This may be facilitated by the unusually large census population size relative to the effective population size. For instance, although racing is the breeding goal, a high proportion (35-50%) of foals born never race and a small proportion enter the breeding population (less than 5% of males and 50% of females). While this selection may purge large-effect mutations it is expected that even at low frequencies, segregation of deleterious alleles within a population with a low effective population size will have consequences for some aspects of population viability and inbreeding depression is likely to be persistent even with ongoing purging.

### Summary of the Invention

According to a first aspect of the present invention, there is provided a method of determining the likelihood of a horse to race, the method comprising the steps of:

(a) identifying at least one biomarker in a sample from the horse;
(b) comparing the at least one biomarker with a respective reference biomarker;

wherein deviation of the at least one biomarker from the respective reference biomarker is indicative that a horse is unlikely to race.

Optionally, the method is a method of determining the likelihood of a horse to race. Further optionally, the method is a method of predicting the likelihood of a horse to race. Still further optionally, the method is a method of identifying a horse that is likely to race.

Optionally, deviation of the at least one biomarker from the respective reference biomarker is indicative that a horse is unlikely to race. Further optionally, deviation of the at least one biomarker from the respective reference biomarker is predictive that a horse is unlikely to race. Still further optionally, deviation of the at least one biomarker from the respective reference biomarker identifies a horse that is unlikely to race.

Optionally, the method is a method of determining the athletic or physical performance or ability of a horse. Further optionally, the method is a method of predicting the athletic or physical performance or ability of a horse. Still further optionally, the method is a method of identifying a horse having athletic or physical performance or ability.

Optionally, deviation of the at least one biomarker from the respective reference biomarker is indicative of poor or less than average athletic or physical performance or ability of a horse. Further optionally, deviation of the at least one biomarker from the respective reference biomarker is predictive of poor or less than average athletic or physical performance or ability of a horse. Still further optionally, deviation of the at least one biomarker from the respective reference biomarker identifies a horse having poor or less than average athletic or physical performance or ability of a horse.

Optionally, the method is a method of determining a trait associated with athletic or physical performance or ability of a horse. Further optionally, the method is a method of identifying a horse having a trait associated with athletic or physical performance or ability of a horse.

Optionally, deviation of the at least one biomarker from the respective reference biomarker is indicative of the presence of a trait associated with athletic or physical performance or ability of a horse. Further optionally, deviation of the at least one biomarker from the respective reference identifies a horse having a trait associated with athletic or physical performance or ability of a horse.

Optionally, the trait is a disease or disorder selected from idiopathic scoliosis, dysfunctional limb formation, limb truncation, polydactyly, acrocallosal syndrome, brain abnormality, cleft palate, brachyspina, reproductive seasonality, compulsive behaviour, deficiency in early osteogenesis, reduction in osteoblast number, β-adrenergic-induced cardiac hypertrophy, dysfunctional birth weight, motor neuron disease; Acrocallosal syndrome, Joubert syndrome 12, Al-Gazali-Bakalinova syndrome, Hydrolethalus syndrome 2, Fanconi anemia, complementation group I, corneal dystrophy, Fuchs endothelial, dysfunctional heart development, dysfunctional bone development, dysfunctional cardiac muscle cell differentiation, dysfunctional cell growth involved in cardiac muscle cell development, dysfunctional adrenergic receptor signalling pathway, dysfunctional adrenergic receptor signalling pathway involved in heart process, dysfunctional lipid transport, dysfunctional regulation of growth, dysfunctional musculoskeletal movement; bone dysfunction, glycogen storage disease, equine polysaccharide storage myopathy, rhabdomyolysis, recurrent exertional rhabdomyolysis, and exertional rhabdomyolysis.

Optionally, the trait is an injury. Further optionally, the trait is a musculoskeletal injury.

Optionally, the method is a method of breeding or mating a horse. Further optionally, the method is a method of identifying a horse for breeding or mating.

Optionally, deviation of the at least one biomarker from the respective reference biomarker is indicative that a horse is unsuitable for breeding or mating. Further optionally, deviation of the at least one biomarker from the respective reference biomarker identifies a horse that is unsuitable for breeding or mating.

Optionally, deviation of the at least one biomarker from the respective reference biomarker is indicative that a horse is unsuitable for breeding or mating with a second horse having the same at least one biomarker deviating from the respective reference biomarker. Further optionally, deviation of the at least one biomarker from the respective reference biomarker identifies a horse that is unsuitable for breeding or mating with a second horse having the same at least one biomarker deviating from the respective reference biomarker.

Optionally, the method further comprises the step of breeding or mating a horse that is suitable for breeding or mating. Further optionally, the method further comprises the step of breeding or mating a horse that is identified as suitable for breeding or mating.

Optionally, the method further comprises the step of breeding or mating a horse that is likely to race, has good or more than average athletic or physical performance or ability, and/or is devoid of a trait associated with athletic or physical performance or ability of a horse.

Optionally, the horse is bred or mated with a second horse that is suitable for breeding or mating, a second horse that is identified as suitable for breeding or mating, and/or a second horse that is likely to race, has good or more than average athletic or physical performance or ability, and/or is devoid of a trait associated with athletic or physical performance or ability of a horse.

Optionally, the or each horse is a thoroughbred horse.

Optionally, the respective reference biomarker is a biomarker in a healthy horse. Still further optionally, the respective reference biomarker is a biomarker in a horse that is likely to race. Still further optionally, the respective reference biomarker is a biomarker in a horse having good or more than average athletic or physical performance or ability of a horse. Still further optionally, the respective reference biomarker is a biomarker in a horse devoid of a trait associated with athletic or physical performance or ability of a horse.

Optionally, the respective reference biomarker is a biomarker in Equus caballus. Further optionally, the respective reference biomarker is a biomarker in a female Equus caballus. Still further optionally, the respective reference biomarker is a biomarker in the reference genome defined by GenBank assembly accession: GCA_002863925.1 and/or RefSeq assembly accession: GCF_002863925.1.

Optionally, the average athletic or physical performance or ability of a horse is the average or mean of the athletic or physical performance or ability of some or all horses in a sample population.

Optionally, the at least one biomarker is a nucleic acid. Further optionally, the at least one biomarker is at least two nucleic acids. Still further optionally, the at least one biomarker is a polynucleotide. Still further optionally, the at least one biomarker is a ribonucleic acid. Still further optionally, the at least one biomarker is a deoxyribonucleic acid.

Optionally, the at least one biomarker is located in a polynucleotide. Still further optionally, the at least one biomarker is located in a ribonucleic acid. Still further optionally, the at least one biomarker is located in a deoxyribonucleic acid.

Optionally, the at least one biomarker is located in a chromosome.

Optionally, the at least one biomarker is located in a chromosome selected from ECA 14, ECA1, ECA3, ECA15, ECA16, and ECA18.

Optionally, the at least one biomarker is located in a chromosome selected from ECA1, ECA3, ECA16.

Optionally, the at least one biomarker is located in a chromosome selected from ECA1, and ECA16.

Optionally, the at least one biomarker is located in chromosome ECA14.

Preferably, the at least one biomarker is located in chromosome ECA14.

Optionally, the at least one biomarker is located within or adjacent a gene.

Optionally, the at least one biomarker is located within or adjacent a gene selected from EFNA5, ACAN, FANCI, TOP2B, SGO1, TAPT1, NKX3-2, SLC1A4, WDPCP, CNOT1, MSTN, PLOD1, KIF7, FANCI, AGBL1, AKAP13, VPS54, OSBPL10; FER, FBXL17; eca-mir-885, and PYGL.

Optionally, the at least one biomarker is located within or adjacent a gene selected from ACAN, EFNA5, FANCI, TOP2B, SGO1, TAPT1, NKX3-2, SLC1A4, WDPCP, CNOT1, MSTN, PLOD1, KIF7, FANCI, AGBL1, AKAP13, VPS54, OSBPL10; FER, and FBXL17.

Optionally, the at least one biomarker is located within or adjacent a gene selected from ACAN, EFNA5, FANCI, TOP2B, SGO1, TAPT1, NKX3-2, SLC1A4, WDPCP, CNOT1, MSTN, PLOD1, KIF7, FANCI, AGBL1, AKAP13, VPS54, and OSBPL10.

Optionally, the at least one biomarker is located within or adjacent a gene selected from KIF7, FANCI, AGBL1, AKAP13, VPS54, and OSBPL10.

Optionally, the at least one biomarker is located within or adjacent a gene selected from ACAN, and FANCI.

Optionally, the at least one biomarker is located within or adjacent a gene selected from TOP2B, and SGO1.

Optionally, the at least one biomarker is located within or adjacent a gene selected from TAPT1, and NKX3-2.

Optionally, the at least one biomarker is located within or adjacent a gene selected from SLC1A4, and WDPCP.

Optionally, the at least one biomarker is located within or adjacent a gene selected from FER, FBXL17, and EFNA5.

Optionally, the at least one biomarker is located within or adjacent a gene selected from eca-mir-885, and PYGL.

Optionally, the at least one biomarker is located within or adjacent the CNOT1 gene.

Optionally, the at least one biomarker is located within or adjacent the MSTN gene.

Optionally, the at least one biomarker is located within or adjacent the PLOD1 gene.

Optionally, the at least one biomarker is located within or adjacent the ACAN gene.

Optionally, the at least one biomarker is located within or adjacent the EFNA5 gene.

Preferably, the at least one biomarker is located within or adjacent the EFNA5 gene.

Optionally, the at least one biomarker is located within or adjacent a gene that is in linkage disequilibrium with a gene described herein.

Optionally, the at least one biomarker is located within or adjacent a gene that is in linkage disequilibrium with one or more gene selected from EFNA5, ACAN, FANCI, TOP2B, SGO1, TAPT1, NKX3-2, SLC1A4, WDPCP, CNOT1, MSTN, PLOD1, KIF7, FANCI, AGBL1, AKAP13, VPS54, OSBPL10; FER, FBXL17; eca-mir-885, and PYGL.

**Table 1. Biomarkers**

| Gene | Description | GenBank (NCBI) Gene ID | Chr | Position (bp) |
|------|-------------|------------------------|-----|---------------|
| ACAN | aggrecan | 100033876 | 1 | 95255522 to 95320938 |
| EFNA5 | ephrin A5 | 100073202 | 14 | 63364569 to 63636038 |
| FANCI | FA complementation group I | 100069712 | 1 | 94927176 to 95017933 |
| TOP2B | DNA topoisomerase II beta | 100059572 | 16 | 58927472 to 59028172 |
| SGO1 | shugoshin 1 | 100051935 | 16 | 63604231 to 63615086 |
| TAPT1 | transmembrane anterior posterior transformation 1 | 100068801 | 3 | 108963273 to 109023472 |
| NKX3-2 | NK3 homeobox 2 | 100069194 | 3 | 111307951 to 111311578 |
| SLC1A4 | solute carrier family 1 member 4 | 100063163 | 15 | 37746505 to 37769073 |
| WDPCP | WD repeat containing planar cell polarity effector | 100063987 | 15 | 38995867 to 39351568 |
| CNOT1 | CCR4-NOT transcription complex subunit 1 | 100052554 | 3 | 11211692 to 11324350 |
| MSTN | Myostatin | 100033832 | 18 | 66605150 to 6610122 |
| PLOD1 | procollagen-lysine,2-oxoglutarate 5-dioxygenase 1 | 100050902 | 2 | 39926928 to 39953350 |
| KIF7 | kinesin family member 7 | 100069672 | 1 | 94648140 to 94663857 |
| AGBL1 | AGBL carboxypeptidase 1 | 100070057 | 1 | 96915356 to 97768843 |
| AKAP13 | A-kinase anchoring protein 13 | 100070242 | 1 | 97988924 to 98311515 |
| VPS54 | VPS54 subunit of GARP complex | 106828367 | 6 | 55004548 to 55111032 |
| OSBPL10 | oxysterol binding protein like 10 | 100057205 | 16 | 53701633 to 53987002 |
| FER | FER tyrosine kinase | 100064643 | 14 | 61998088 to 62440170 |
| FBXL17 | F-box and leucine rich repeat protein 17 | 100146140 | 14 | 62732148 to 63195702 |
| eca-mir-885 | non-coding RNA | 100315068 | 16 | 8,057,788 to 8,057,861 |
| PYGL | glycogen phosphorylase L | 100066726 | 1 | 186995497 to 187024683 |

Optionally, the at least one biomarker is a mutation.

Optionally, the at least one biomarker is a mutation selected from an insertion, a deletion, and a substitution.

Optionally, the mutation is selected from an in frame mutation, a splice site mutation, a frameshift mutation, a transition mutation, a transversion mutation, a synonymous substitution mutation, a nonsynonymous substitution mutation, a missense mutation, and a nonsense mutation.

Optionally, the at least one biomarker is a point mutation.

Optionally, the at least one biomarker is a single nucleotide polymorphism (SNP).

Optionally, the at least one biomarker is located at a position (bp) as defined in the reference genome defined by GenBank assembly accession: GCA_002863925.1 and/or RefSeq assembly accession: GCF_002863925.1.

Optionally, the at least one biomarker is located at a position (bp) of the chromosome ECA14 from 63424953 to 64382244.

Optionally, the at least one biomarker is located at a position (bp) of the chromosome ECA14 selected from:63883487, 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244.

Optionally, the at least one biomarker is located at a position (bp) of the chromosome ECA14 selected from:63883487, 63424953, 63476246, 63518338, 63562113, 63599074, 63645752, 63682826, 63684190, 63688573, 63701442, 63709333, 63717804, 63825182, 63848624, 63850407, 63909899, 63933713, 63972605, 63975161, 63987230, 64000803, 64028113, 64080217, 64090380, 64133314, 64134277, and 64184542.

Optionally, the at least one biomarker is located at position 63883487bp of the chromosome ECA14.

Preferably, the at least one biomarker is located at position 63883487bp of the chromosome ECA14.

Optionally, the at least one biomarker is a single nucleotide polymorphism having a reference SNP cluster ID selected from: rs1144708552, rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the at least one biomarker is a single nucleotide polymorphism having a reference SNP cluster ID selected from: rs1144708552, rs395101612, rs68958215, rs68958240, rs1148244706, rs1147391875, rs1151500701, rs394409489, rs394937599, rs396647223, rs68959726, rs1144890403, rs1138630473, rs397243755, rs396356157, rs396601898, rs68991720, rs397331375, rs1140055494, rs1137832255, rs1136163000, rs1139936607, rs1152140905, rs68991774, rs68991779, rs1138384269, rs68993404, and rs1148269508.

Optionally, the at least one biomarker is a single nucleotide polymorphism having a reference SNP cluster ID 1144708552 (rs1144708552).

Optionally, the at least one biomarker is located at a position (bp) of the chromosome ECA1 from 95255522 to 187024683.

Optionally, the at least one biomarker is located at a position (bp) of the chromosome ECA1 from 96704107 to 97202669.

Optionally, the at least one biomarker is located at a position (bp) of the chromosome ECA1 from 95255522 to 95320938.

Optionally, the at least one biomarker is located at a position (bp) of the chromosome ECA1 from 186995497 to 187024683.

Optionally, the at least one biomarker is located at a position (bp) of the chromosome ECA1 selected from:95270484, 95271115, 95271452, 95272282, 95272998, 95273021, 95273215, 95273323, 95273484, 95274059, 95274276, 95274281, 95274294, 95274300, 95274303, 95274430, 95274878, 95284467, 95284551, 95293801; and 186974288.

Optionally, the at least one biomarker is located at a position (bp) of the chromosome ECA1 selected from: 96704107 and 96719990.

Optionally, the at least one biomarker is located at position 96704107bp of the chromosome ECA1.

Optionally, the at least one biomarker is located at position 96719990bp of the chromosome ECA1.

Optionally, the at least one biomarker is located at position 186974288bp of the chromosome ECA1.

Optionally, the at least one biomarker is a single nucleotide polymorphism having a reference SNP cluster ID 68492263 (rs68492263).

Optionally, the at least one biomarker is a single nucleotide polymorphism having a reference SNP cluster ID 1143204553 (rs1143204553).

Optionally, the at least one biomarker is located at a position (bp) of the chromosome ECA16 from 50903024 to 66954937.

Optionally, the at least one biomarker is located at a position (bp) of the chromosome ECA16 from 54707379 to 66954937.

Optionally, the at least one biomarker is located at a position (bp) of the chromosome ECA16 from 50903024 to 53259316.

Optionally, the at least one biomarker is located at a position (bp) of the chromosome ECA16 from 58927472 to 59028172.

Optionally, the at least one biomarker is located at a position (bp) of the chromosome ECA16 from 8,057,788 to 8,057,861.

Optionally, the at least one biomarker is located at a position (bp) of the chromosome ECA16 selected from: 61350408, 61316728, 61485318, 62361235, 61881224, 62359700, 61579357, 61888299, 61282867, 61955520, 61980497, 51763459, 62173781, 52114617, 61851562, 51769224; and 8037334.

Optionally, the at least one biomarker is located at a position (bp) of the chromosome ECA16 selected from: 61350408 and 51763459.

Optionally, the at least one biomarker is located at position 61350408bp of the chromosome ECA16.

Optionally, the at least one biomarker is located at position 51763459bp of the chromosome ECA16.

Optionally, the at least one biomarker is located at position 8037334bp of the chromosome ECA16.

Optionally, the at least one biomarker is a single nucleotide polymorphism having a reference SNP cluster ID selected from: rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; and rs69045681.

Optionally, the at least one biomarker is a single nucleotide polymorphism having a reference SNP cluster ID selected from: 1136876668 (rs1136876668) and 394055886 (rs394055886).

Optionally, the at least one biomarker is a single nucleotide polymorphism having a reference SNP cluster ID 1136876668 (rs1136876668).

Optionally, the at least one biomarker is a single nucleotide polymorphism having a reference SNP cluster ID 394055886 (rs394055886).

Optionally, the at least one biomarker is a single nucleotide polymorphism having a reference SNP cluster ID 69045681 (rs69045681).

Optionally, the at least one biomarker is located at a position (bp) of the chromosome ECA3 from 108963273 to 14427200.

Optionally, the at least one biomarker is located at a position (bp) of the chromosome ECA3 from 109434554 to 111674968.

Optionally, the at least one biomarker is located at a position (bp) of the chromosome ECA3 from 12330293 to 14427200.

Optionally, the at least one biomarker is located at a position (bp) of the chromosome ECA3 from 108963273 to 109023472.

Optionally, the at least one biomarker is located at a position (bp) of the chromosome ECA3 from 111307951 to 111311578.

Optionally, the at least one biomarker is located at a position (bp) of the chromosome ECA3 selected from: 110575172, 110626960, 110580051, 13600864; 110384214, 110905597, 13451508, 110890960; 13940199, 110738405, and 13668298.

Optionally, the at least one biomarker is located at a position (bp) of the chromosome ECA3 selected from: 110575172bp and 13600864bp of the chromosome ECA3.

Optionally, the at least one biomarker is located at position 110575172bp of the chromosome ECA3.

Optionally, the at least one biomarker is located at position 13600864bp of the chromosome ECA3.

Optionally, the at least one biomarker is a single nucleotide polymorphism having a reference SNP cluster ID selected from: rs1142345000, rs1143627458, rs1151161986, rs1138851722, rs1141842338, rs1144608958, rs395438793, rs1137710709, rs396437371, rs1143815205, and rs1148874071.

Optionally, the at least one biomarker is a single nucleotide polymorphism having a reference SNP cluster ID selected from: 1142345000 (rs1142345000) and 1138851722 (rs1138851722).

Optionally, the at least one biomarker is a single nucleotide polymorphism having a reference SNP cluster ID 1142345000 (rs1142345000).

Optionally, the at least one biomarker is a single nucleotide polymorphism having a reference SNP cluster ID 1138851722 (rs1138851722).

Optionally, the at least one biomarker is located at a position (bp) of the chromosome ECA15 from 34836277 to 37696834.

Optionally, the at least one biomarker is located at position 37623615bp of the chromosome ECA15.

Optionally, the at least one biomarker is a single nucleotide polymorphism having a reference SNP cluster ID 1144264641 (rs1144264641).

Optionally, the at least one biomarker is located at a position (bp) of the chromosome ECA18 from 68395895 to 69051761.

Optionally, the at least one biomarker is located at position 68535533bp of the chromosome ECA18.

Optionally, the at least one biomarker is a single nucleotide polymorphism having a reference SNP cluster ID 1140720421 (rs1140720421).

Optionally, the at least one biomarker is a single nucleotide polymorphism selected from: rs1144708552; rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; rs1140720421; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the at least one biomarker is a single nucleotide polymorphism selected from: rs1144708552; rs68492263; rs1136876668, rs1142345000, rs1144643078, rs394291305, rs1144152381, rs1138954915, rs1143627458, rs1144264641, rs1139578484, rs1141586753, rs396933426, rs1149103864, rs1151161986, rs1148520935, rs1138851722, rs1140720421, rs1141842338, rs1149520510, rs1144608958, rs395438793, rs394055886, rs1137710709, rs1145156936, rs395900184, rs396437371, rs1143815205, rs1137740805, rs1143089369, rs1148874071; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the at least one biomarker is a single nucleotide polymorphism selected from: rs1144708552; rs68492263; rs1136876668, rs394055886; rs1142345000, rs1138851722; rs1144264641; rs1140720421; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the at least one biomarker is a single nucleotide polymorphism selected from: rs1144708552; rs68492263; rs1136876668; rs394055886; rs1142345000; rs1144264641; rs1140720421; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the at least one biomarker is a single nucleotide polymorphism selected from: rs1144708552; rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; and rs1140720421.

Optionally, the at least one biomarker is a single nucleotide polymorphism selected from: rs1144708552; rs68492263; rs1136876668, rs1142345000, rs1144643078, rs394291305, rs1144152381, rs1138954915, rs1143627458, rs1144264641, rs1139578484, rs1141586753, rs396933426, rs1149103864, rs1151161986, rs1148520935, rs1138851722, rs1140720421, rs1141842338, rs1149520510, rs1144608958, rs395438793, rs394055886, rs1137710709, rs1145156936, rs395900184, rs396437371, rs1143815205, rs1137740805, rs1143089369, and rs1148874071.

Optionally, the at least one biomarker is a single nucleotide polymorphism selected from: rs1144708552; rs68492263; rs1136876668, rs394055886; rs1142345000, rs1138851722; rs1144264641; and rs1140720421.

Optionally, the at least one biomarker is a single nucleotide polymorphism selected from: rs68492263; rs1136876668; rs394055886; rs1142345000; rs1144264641; and rs1140720421.

Optionally, the at least one biomarker is the single nucleotide polymorphism rs1144708552.

Preferably, the at least one biomarker is the single nucleotide polymorphism rs1144708552.

Optionally, the presence of a homozygous single nucleotide polymorphism is indicative that a horse is unlikely to race.

Optionally, the presence of a homozygous single nucleotide polymorphism is indicative that a horse has poor or less than average athletic or physical performance or ability and/or has a trait associated with athletic or physical performance or ability of a horse.

Optionally, the at least one biomarker is a single nucleotide polymorphism in linkage disequilibrium with a single nucleotide polymorphism described herein.

Optionally, the at least one biomarker is a single nucleotide polymorphism in linkage disequilibrium with a single nucleotide polymorphism selected from and one or more of: rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; rs1140720421; rs1144708552; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism is located at a position (bp) of the chromosome ECA14 from 63424953 to 64382244.

Optionally, the single nucleotide polymorphism is located at a position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244.

Optionally, the single nucleotide polymorphism is located at a position (bp) of the chromosome ECA14 selected from: 63424953, 63476246, 63518338, 63562113, 63599074, 63645752, 63682826, 63684190, 63688573, 63701442, 63709333, 63717804, 63825182, 63848624, 63850407, 63883487, 63909899, 63933713, 63972605, 63975161, 63987230, 64000803, 64028113, 64080217, 64090380, 64133314, 64134277, and 64184542.

Optionally, the single nucleotide polymorphism is located at position 63883487bp of the chromosome ECA14.

Optionally, the single nucleotide polymorphism has a reference SNP cluster ID selected from: rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism has a reference SNP cluster ID selected from: rs395101612, rs68958215, rs68958240, rs1148244706, rs1147391875, rs1151500701, rs394409489, rs394937599, rs396647223, rs68959726, rs1144890403, rs1138630473, rs397243755, rs396356157, rs396601898, rs1144708552, rs68991720, rs397331375, rs1140055494, rs1137832255, rs1136163000, rs1139936607, rs1152140905, rs68991774, rs68991779, rs1138384269, rs68993404, and rs1148269508.

Optionally, the single nucleotide polymorphism has a reference SNP cluster ID 1144708552 (rs1144708552).

Optionally, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from a nucleic acid sequence selected from any one of:

(1)

TGGACCGAGACAAACAGATTGGTTCTAATATGAGTCGGGTTCGCAGACAGATTGTGAGC AAAGGGACATGCTCTCCTTTGGACT;

(2)

TGAACCGAGACAAACAGATTGGCCTTAATATAAACTGGGTCAGCAGACAGATTGTGAACA

AAGGGGTACATCATTCTTTGGACT;

(3)

CGGGCCGGGAAAGACAAACCTGCCTTAATATAAACTAGGTCCATGGGCGAATTATAGGA

GAAGGGACGTGCTCTCCTTTGGACT;

(4)

CGGACCGAGACAAAAGATTGGCCTCGGCAGACGTCGTGCCCACAGACAAGTTATGAG

AAAGAGAACACACCATTTCCGGAACT;

(5)

TGGACCAAGACAAACAGATTGGTTCTGGCAGAAGCCGGGTCCATAGATAAACCGGGGG

AGGAAAGACACACCATCCTTTTGACT;

(6)

TGGAATGATGAGGGCCGGCCTGTTCTGGTGTACGTCGGATCCATAAACGGACCGGGAG

AAAGAGAACACACCATTTCCGGAACT;

(7)

TAGAATGATGAGGGCCGGCCTATTCTAATATGAGTCGGATCAGCAGACAGATTGTAGGA

GAAGGGACGTGCTCTCTTCTGGACT;

(8)

TGGACCGAGACAAAAGATTGGCCTCGGCAGACGTCGGGTCCATAGATAAACCGGGGG

AGGAAAGACATGTTAGTTCCTGACTC; and

(9)

CGGGCCGGGAAAGACAAACCTATTCTAATATGAGTCGGATCAGCAGACAGATTGTAGGA

GAAGGGACGCACCATTTCCGGAACT.

Optionally, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from the nucleic acid sequence (3)

CGGGCCGGGAAAGACAAACCTGCCTTAATATAAACTAGGTCCATGGGCGAATTATAGGAGAAGG GACGTGCTCTCCTTTGGACT.

Preferably, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from the nucleic acid sequence (3) CGGGCCGGGAAAGACAAACCTGCCTTAATATAAACTAGGTCCATGGGCGAATTATAGGAGAAGG GACGTGCTCTCCTTTGGACT.

Optionally, the nucleotide located at each position (bp) of the chromosome ECA14 is the nucleotide located at the respective nucleic acid position of the nucleic acid sequence selected from any one of SEQ ID NOs 1-9.

Optionally, the nucleotide located at each position (bp) of the chromosome ECA14 is the nucleotide located at the respective nucleic acid position of the nucleic acid sequence defined by SEQ ID NO 3.

Optionally, the nucleotide located at each position (bp) of the chromosome ECA14 is the respective nucleotide located at the respective nucleic acid position of the nucleic acid sequence selected from any one of SEQ ID NOs 1-9.

Optionally, the nucleotide located at each position (bp) of the chromosome ECA14 is the respective nucleotide located at the respective nucleic acid position of the nucleic acid sequence defined by SEQ ID NO 3.

Optionally, the nucleotide located at each position (bp) of the chromosome ECA14 is the respective nucleotide of a haplotype having the nucleic acid sequence selected from any one of SEQ ID NOs 1-9.

Optionally, the nucleotide located at each position (bp) of the chromosome ECA14 is the respective nucleotide of a haplotype having the nucleic acid sequence defined by SEQ ID NO 3.

Optionally, the horse is a horse from Europe, Australia, New Zealand, the USA, Japan, or South Africa. Optionally, the horse is a horse born in Europe, Australia, New Zealand, the USA, Japan, or South Africa.

Optionally, the horse is a horse from Europe, Australia, or New Zealand. Optionally, the horse is a horse born in Europe, Australia, or New Zealand.

Optionally, the horse is a horse from the USA. Optionally, the horse is a horse born in the USA.

Preferably, the horse is a horse from Europe. More preferably, the horse is a horse born in Europe.

Optionally, the horse is a male horse. Alternatively, the horse is a female horse.

Optionally, the horse is less than one year old. Further optionally, the horse is less than 20 months old. Still further optionally, the horse is less than two years old. Still further optionally, the horse is less than three years old. Still further optionally, the horse is less than four years old. Still further optionally, the horse is less than five years old. Still further optionally, the horse is less than six years old.

Preferably, the horse is less than five years old.

According to a second aspect of the present invention, there is provided a method of determining the likelihood of a horse to race, the method comprising the steps of:

(a) identifying at least one run of homozygosity;
(b) determining the length of the at least one run of homozygosity;
(c) determining the length of a genome; and
(d) comparing the length of the at least one run of homozygosity to the length of the genome;

wherein a ratio of the length of the at least one run of homozygosity compared to the length of the genome is indicative that a horse is likely to race.

Optionally, the method is a method of determining the likelihood of a horse to race. Further optionally, the method is a method of predicting the likelihood of a horse to race. Still further optionally, the method is a method of identifying a horse that is likely to race.

Optionally, a ratio of the length of the at least one run of homozygosity compared to the length of the genome is indicative that a horse is likely to race. Further optionally, a ratio of the length of the at least one run of homozygosity compared to the length of the genome is predictive that a horse is likely to race. Still further optionally, a ratio of the length of the at least one run of homozygosity compared to the length of the genome identifies a horse that is likely to race.

Optionally, the method is a method of determining the athletic or physical performance or ability of a horse. Further optionally, the method is a method of predicting the athletic or physical performance or ability of a horse. Still further optionally, the method is a method of identifying a horse having athletic or physical performance or ability.

Optionally, a ratio of the length of the at least one run of homozygosity compared to the length of the genome is indicative of good or more than average athletic or physical performance or ability of a horse. Further optionally, a ratio of the length of the at least one run of homozygosity compared to the length of the genome is predictive of good or more than average athletic or physical performance or ability of a horse. Still further optionally, a ratio of the length of the at least one run of homozygosity compared to the length of the genome identifies a horse having good or more than average athletic or physical performance or ability of a horse.

Optionally, the method is a method of determining a trait associated with athletic or physical performance or ability of a horse. Further optionally, the method is a method of identifying a horse having a trait associated with athletic or physical performance or ability of a horse.

Optionally, a ratio of the length of the at least one run of homozygosity compared to the length of the genome is indicative of the absence of a trait associated with athletic or physical performance or ability of a horse. Further optionally, a ratio of the length of the at least one run of homozygosity compared to the length of the genome identifies a horse devoid of a trait associated with athletic or physical performance or ability of a horse.

Optionally, the trait is a disease or disorder selected from idiopathic scoliosis, dysfunctional limb formation, limb truncation, polydactyly, acrocallosal syndrome, brain abnormality, cleft palate, brachyspina, reproductive seasonality, compulsive behaviour, deficiency in early osteogenesis, reduction in osteoblast number, $\beta$-adrenergic-induced cardiac hypertrophy, dysfunctional birth weight, motor neuron disease; Acrocallosal syndrome, Joubert syndrome 12, Al-Gazali-Bakalinova syndrome, Hydrolethalus syndrome 2, Fanconi anemia, complementation group I, corneal dystrophy, Fuchs endothelial, dysfunctional heart development, dysfunctional bone development, dysfunctional cardiac muscle cell differentiation, dysfunctional cell growth involved in cardiac muscle cell development, dysfunctional adrenergic receptor signalling pathway, dysfunctional adrenergic receptor signalling pathway involved in heart process, dysfunctional lipid transport, dysfunctional regulation of growth, dysfunctional musculoskeletal movement; bone dysfunction, glycogen storage disease, equine polysaccharide storage myopathy, rhabdomyolysis, recurrent exertional rhabdomyolysis, and exertional rhabdomyolysis.

Optionally, the trait is an injury. Further optionally, the trait is a musculoskeletal injury.

Optionally, the method is a method of breeding or mating a horse. Further optionally, the method is a method of identifying a horse for breeding or mating.

Optionally, a ratio of the length of the at least one run of homozygosity compared to the length of the genome is indicative that a horse is suitable for breeding or mating. Further optionally, a ratio of the length of the at least one run of homozygosity compared to the length of the genome identifies a horse that is suitable for breeding or mating.

Optionally, the method further comprises the step of breeding or mating a horse that is suitable for breeding or mating. Further optionally, the method further comprises the step of breeding or mating a horse that is identified as suitable for breeding or mating.

Optionally, the method further comprises the step of breeding or mating a horse that is likely to race, has good or more than average athletic or physical performance or ability, and/or is devoid of a trait associated with athletic or physical performance or ability of a horse.

Optionally, the horse is bred or mated with a second horse that is suitable for breeding or mating, a second horse that is identified as suitable for breeding or mating, and/or a second horse that is likely to race, has good or more than average athletic or physical performance or ability, and/or is devoid of a trait associated with athletic or physical performance or ability of a horse.

Optionally, the or each horse is a thoroughbred horse.

Optionally, a ratio of the length of the at least one run of homozygosity compared to the length of the genome of <0.50 is indicative that a horse is likely to race. Further optionally, a ratio of the length of the at least one run of homozygosity compared to the length of the genome of <0.40 is indicative that a horse is likely to race.

Optionally, a ratio of the length of the at least one run of homozygosity compared to the length of the genome of <0.40, optionally <0.35, optionally <0.30, optionally <0.29, optionally <0.28, optionally <0.27, optionally <0.26, optionally <0.25, optionally <0.24, optionally <0.23, optionally <0.22, optionally <0.21, optionally <0.20, optionally <0.19, optionally <0.18, optionally <0.17, optionally <0.16, optionally <0.15, optionally <0.1 is indicative that a horse is likely to race.

Optionally, the identifying step comprises identifying at least one segment of a genome.

Optionally, the identifying step comprises identifying at least one segment of at least one polynucleotide. Further optionally, the identifying step comprises identifying at least one segment of two polynucleotides. Still further optionally, the identifying step comprises identifying at least one segment of two complementary polynucleotides. Still further optionally, the identifying step comprises identifying at least one segment of first and second complementary polynucleotides.

Optionally, the polynucleotide is a nucleic acid. Further optionally, the polynucleotide is a ribonucleic acid. Still further optionally, the polynucleotide is a deoxyribonucleic acid.

Optionally, the identifying step comprises identifying at least one segment comprising at least one gene. Further optionally, the identifying step comprises identifying at least one segment comprising two genes. Still further optionally, the identifying step comprises identifying at least one segment comprising two complementary genes. Still further optionally, the identifying step comprises identifying at least one segment comprising first and second complementary genes.

Optionally, the identifying step comprises identifying at least one segment comprising at least one allele. Further optionally, the identifying step comprises identifying at least one segment comprising two alleles. Still further optionally, the identifying step comprises identifying at least one segment comprising two complementary alleles. Still further optionally, the identifying step comprises identifying at least one segment comprising first and second complementary alleles.

Optionally, the identifying step comprises identifying at least one segment comprising two homozygous alleles. Still further optionally, the identifying step comprises identifying at least one segment comprising two homozygous alleles. Still further optionally, the identifying step comprises identifying at least one segment comprising first and second homozygous alleles.

Optionally, the identifying step comprises identifying at least one segment comprising the same alleles. Still further optionally, the identifying step comprises identifying at least one segment comprising the same two alleles. Still further optionally, the identifying step comprises identifying at least one segment comprising the same first and second alleles.

Optionally, the determining the length of the at least one run of homozygosity step comprises determining the number of nucleotides in the at least one run of homozygosity. Further optionally, the determining the length of the at least one run of homozygosity step comprises determining the number of nucleotides in the at least one segment.

Optionally, the determining the length of the at least one run of homozygosity step comprises determining the number of nucleotides in all runs of homozygosity. Further optionally, the determining the length of the at least one run of homozygosity step comprises determining the number of nucleotides in all segments.

Optionally, determining the length of a genome comprises determining the number of nucleotides in the genome.

Optionally, the genome comprises 2,281,000,000 nucleotides.

Optionally, determining the length of a genome comprises determining the length of the autosomal genome. Further optionally, determining the length of the autosomal genome comprises determining the number of nucleotides in the autosomal genome.

Optionally, the autosomal genome is the genome of Equus caballus. Further optionally, the autosomal genome is the genome of a female Equus caballus. Still further optionally, the autosomal genome is the reference genome defined by GenBank assembly accession: GCA_002863925.1 and/or RefSeq assembly accession: GCF_002863925.1.

Optionally, the autosomal genome comprises 2,281,000,000 nucleotides.

Optionally, determining the length of a genome comprises determining the length of the genome defined by autosomal chromosomes. Further optionally, determining the length of the autosomal genome comprises determining the number of nucleotides in the genome defined by autosomal chromosomes.

Optionally, the genome defined by autosomal chromosomes comprises 2,281,000,000 nucleotides.

Optionally, determining the length of a genome comprises determining the length of the genome defined by chromosomes 1-31. Further optionally, determining the length of the autosomal genome comprises determining the number of nucleotides in the genome defined by chromosomes 1-31.

Optionally, determining the length of a genome comprises determining the length of the genome defined by chromosomes 1-31 of the reference genome defined by GenBank assembly accession: GCA_002863925.1 and/or RefSeq assembly accession: GCF_002863925.1. Further optionally, determining the length of the autosomal genome comprises determining the number of nucleotides in the genome defined by chromosomes 1-31 of the reference genome defined by GenBank assembly accession: GCA_002863925.1 and/or RefSeq assembly accession: GCF_002863925.1.

Optionally, the genome defined by chromosomes 1-31 comprises 2,281,000,000 nucleotides.

Optionally, the method comprises the further step of comparing the ratio of the length of the at least one run of homozygosity compared to the length of the autosomal genome to a reference ratio.

Optionally, the reference ratio is the average or mean of the ratios of the length of the at least one run of homozygosity compared to the length of the autosomal genome of some or all horses in a sample population.

Optionally, the reference ratio is <0.50. Further optionally, the reference ratio is <0.40. Still further optionally, the reference ration is <0.35, optionally <0.30, optionally <0.29, optionally <0.28, optionally <0.27, optionally <0.26, optionally <0.25, optionally <0.24, optionally <0.23, optionally <0.22, optionally <0.21, optionally <0.20, optionally <0.19, optionally <0.18, optionally <0.17, optionally <0.16, optionally <0.15, optionally <0.1.

Optionally, the reference ratio is 0.50. Further optionally, the reference ratio is 0.40. Still further optionally, the reference ratio is 0.35, optionally 0.30, optionally 0.29, optionally 0.28, optionally 0.27, optionally 0.26, optionally 0.25, optionally 0.24, optionally 0.23, optionally 0.22, optionally 0.21, optionally 0.20, optionally 0.19, optionally 0.18, optionally 0.17, optionally 0.16, optionally 0.15, optionally 0.1.

Optionally, an increase in the ratio compared to the reference ratio is indicative that a horse is less likely to race.

Optionally, a decrease in the ratio compared to the reference ratio is indicative that a horse is more likely to race.

Optionally, an increase of at least 5% in the ratio of length of the at least one run of homozygosity compared to a length of the autosomal genome is indicative that a horse is at least to race.

Optionally, an increase of at least 2%, optionally at least 3%, optionally at least 4%, optionally at least 5%, optionally at least 6%, optionally at least 7%, optionally at least 8%, optionally at least 9%, optionally at least 10%, optionally at least 15% in the ratio of length of the at least one run of homozygosity compared to a length of the autosomal genome is indicative that a horse is unlikely to race.

Optionally, an increase of at least 5% in the ratio of length of the at least one run of homozygosity compared to a length of the autosomal genome is indicative that a horse is at least 88.5% likely to race.

Optionally, an increase of at least 10% in the ratio of length of the at least one run of homozygosity compared to a length of the autosomal genome is indicative that a horse is 44% less likely to race.

Optionally, the horse is a horse from Europe, Australia, New Zealand, the USA, Japan, or South Africa. Optionally, the horse is a horse born in Europe, Australia, New Zealand, the USA, Japan, or South Africa.

Optionally, the horse is a horse from Europe, Australia, or New Zealand. Optionally, the horse is a horse born in Europe, Australia, or New Zealand.

Optionally, the horse is a horse from the USA. Optionally, the horse is a horse born in the USA.

Preferably, the horse is a horse from Europe. More preferably, the horse is a horse born in Europe.

Optionally, the horse is a male horse. Alternatively, the horse is a female horse.

Optionally, the horse is less than one year old. Further optionally, the horse is less than 20 months old. Still further optionally, the horse is less than two years old. Still further optionally, the horse is less than three years old. Still further optionally, the horse is less than four years old. Still further optionally, the horse is less than five years old. Still further optionally, the horse is less than six years old.

Preferably, the horse is less than five years old.

Optionally, the run of homozygosity has a minimum length of 100 kb. Further optionally, the run of homozygosity has a minimum length of 200 kb. Still further optionally, the run of homozygosity has a minimum length of 300 kb. Still further optionally, the run of homozygosity has a minimum length of 400 kb.

Preferably, the run of homozygosity has a minimum length of 300 kb.

Optionally, the run of homozygosity has a minimum length of 5 Mb. Alternatively, the run of homozygosity has a maximum length of 5 Mb.

Preferably, the run of homozygosity has a minimum length of 5 Mb.

Optionally, the horse is a horse from Europe or a horse born in Europe, and the run of homozygosity has a maximum length of 5 Mb.

Alternatively, the horse is a horse from Australia or New Zealand or a horse born in Australia or New Zealand, and the run of homozygosity has a minimum length of 5 Mb.

Optionally, the identifying step comprises identifying at least one segment of a genome comprising the at least one single nucleotide polymorphism.

Optionally, the identifying step comprises identifying at least one segment of at least one polynucleotide comprising the at least one single nucleotide polymorphism.

Optionally, the polynucleotide is a nucleic acid. Further optionally, the polynucleotide is a ribonucleic acid. Still further optionally, the polynucleotide is a deoxyribonucleic acid.

Optionally, the identifying step comprises identifying at least one segment comprising at least one single nucleotide polymorphism. Further optionally, the identifying step comprises identifying at least one segment comprising at least one homozygous single nucleotide polymorphism.

Optionally, the single nucleotide polymorphism is selected from: rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; rs1140720421; rs1144708552; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism is selected from: rs68492263; rs1136876668, rs1142345000, rs1144643078, rs394291305, rs1144152381, rs1138954915, rs1143627458, rs1144264641, rs1139578484, rs1141586753, rs396933426, rs1149103864, rs1151161986, rs1148520935, rs1138851722, rs1140720421, rs1141842338, rs1149520510, rs1144608958, rs395438793, rs394055886, rs1137710709, rs1145156936, rs395900184, rs396437371, rs1143815205, rs1137740805, rs1143089369, rs1148874071; rs1144708552 rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism is selected from: rs68492263; rs1136876668, rs394055886; rs1142345000, rs1138851722; rs1144264641; rs1140720421; rs1144708552 rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism is selected from: rs68492263; rs1136876668; rs394055886; rs1142345000; rs1144264641; rs1140720421; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism is selected from: rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; rs1140720421; and rs1144708552.

Optionally, the single nucleotide polymorphism is selected from: rs68492263; rs1136876668, rs1142345000, rs1144643078, rs394291305, rs1144152381, rs1138954915, rs1143627458, rs1144264641, rs1139578484, rs1141586753, rs396933426, rs1149103864, rs1151161986, rs1148520935, rs1138851722, rs1140720421, rs1141842338, rs1149520510, rs1144608958, rs395438793, rs394055886, rs1137710709, rs1145156936, rs395900184, rs396437371, rs1143815205, rs1137740805, rs1143089369, rs1148874071; and rs1144708552.

Optionally, the single nucleotide polymorphism is selected from: rs68492263; rs1136876668, rs394055886; rs1142345000, rs1138851722; rs1144264641; rs1140720421; and rs1144708552.

Optionally, the single nucleotide polymorphism is selected from: rs68492263; rs1136876668; rs394055886; rs1142345000; rs1144264641; and rs1140720421.

Optionally, the single nucleotide polymorphism is rs1144708552.

Optionally, the single nucleotide polymorphism is located at a position (bp) of the chromosome ECA14 from 63424953 to 64382244.

Optionally, the single nucleotide polymorphism is located at a position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244.

Optionally, the single nucleotide polymorphism is located at a position (bp) of the chromosome ECA14 selected from: 63424953, 63476246, 63518338, 63562113, 63599074, 63645752, 63682826, 63684190, 63688573, 63701442, 63709333, 63717804, 63825182, 63848624, 63850407, 63883487, 63909899, 63933713, 63972605, 63975161, 63987230, 64000803, 64028113, 64080217, 64090380, 64133314, 64134277, and 64184542.

Optionally, the single nucleotide polymorphism is located at position 63883487bp of the chromosome ECA14.

Optionally, the single nucleotide polymorphism has a reference SNP cluster ID selected from: rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1144708552, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

Optionally, the single nucleotide polymorphism has a reference SNP cluster ID selected from: rs395101612, rs68958215, rs68958240, rs1148244706, rs1147391875, rs1151500701, rs394409489, rs394937599, rs396647223, rs68959726, rs1144890403, rs1138630473, rs397243755, rs396356157, rs396601898, rs1144708552, rs68991720, rs397331375, rs1140055494, rs1137832255, rs1136163000, rs1139936607, rs1152140905, rs68991774, rs68991779, rs1138384269, rs68993404, and rs1148269508.

Optionally, the single nucleotide polymorphism has a reference SNP cluster ID 1144708552 (rs1144708552).

Optionally, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from a nucleic acid sequence selected from any one of:

(1)

TGGACCGAGACAAACAGATTGGTTCTAATATGAGTCGGGTTCGCAGACAGATTGTGAGC AAAGGGACATGCTCTCCTTTGGACT;

(2)

TGAACCGAGACAAACAGATTGGCCTTAATATAAACTGGGTCAGCAGACAGATTGTGAACA AAGGGGTACATCATTCTTTGGACT;

(3)

CGGGCCGGGAAAGACAAACCTGCCTTAATATAAACTAGGTCCATGGGCGAATTATAGGA GAAGGGACGTGCTCTCCTTTGGACT;

(4)

CGGACCGAGACAAAAAGATTGGCCTCGGCAGACGTCGTGCCCACAGACAAGTTATGAG AAAGAGAACACACCATTTCCGGAACT;

(5)

TGGACCAAGACAAACAGATTGGTTCTGGCAGAAGCCGGGTCCATAGATAAACCGGGGG AGGAAAGACACACCATCCTTTTGACT;

(6)

TGGAATGATGAGGGCCGGCCTGTTCTGGTGTACGTCGGATCCATAAACGGACCGGGAG AAAGAGAACACACCATTTCCGGAACT;

(7)

TAGAATGATGAGGGCCGGCCTATTCTAATATGAGTCGGATCAGCAGACAGATTGTAGGA GAAGGGACGTGCTCTCTTCTGGACT;

(8)

TGGACCGAGACAAAAAGATTGGCCTCGGCAGACGTCGGGTCCATAGATAAACCGGGGG AGGAAAGACATGTTAGTTCCTGACTC; and

(9)

CGGGCCGGGAAAGACAAACCTATTCTAATATGAGTCGGATCAGCAGACAGATTGTAGGA GAAGGGACGCACCATTTCCGGAACT.

Optionally, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307,   64358002,   and   64382244   is   a   nucleic   acid   selected   from   the   nucleic   acid   sequence   (3) CGGGCCGGGAAAGACAAACCTGCCTTAATATAAACTAGGTCCATGGGCGAATTATAGGAGAAGG        GACGTGCTCTCCTTTGGACT.

Preferably, the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from the nucleic acid sequence (3) CGGGCCGGGAAAGACAAACCTGCCTTAATATAAACTAGGTCCATGGGCGAATTATAGGAGAAGG GACGTGCTCTCCTTTGGACT.

Optionally, the nucleotide located at each position (bp) of the chromosome ECA14 is the nucleotide located at the respective nucleic acid position of the nucleic acid sequence selected from any one of SEQ ID NOs 1-9.

Optionally, the nucleotide located at each position (bp) of the chromosome ECA14 is the nucleotide located at the respective nucleic acid position of the nucleic acid sequence defined by SEQ ID NO 3.

Optionally, the nucleotide located at each position (bp) of the chromosome ECA14 is the respective nucleotide located at the respective nucleic acid position of the nucleic acid sequence selected from any one of SEQ ID NOs 1-9.

Optionally, the nucleotide located at each position (bp) of the chromosome ECA14 is the respective nucleotide located at the respective nucleic acid position of the nucleic acid sequence defined by SEQ ID NO 3.

Optionally, the nucleotide located at each position (bp) of the chromosome ECA14 is the respective nucleotide of a haplotype having the nucleic acid sequence selected from any one of SEQ ID NOs 1-9.

Optionally, the nucleotide located at each position (bp) of the chromosome ECA14 is the respective nucleotide of a haplotype having the nucleic acid sequence defined by SEQ ID NO 3.

**Brief description of the drawings**

The present invention will now be described with reference to the accompanying drawings and appended non-limiting examples in which:

**Figure 1** illustrates predicted probability (and 95% confidence intervals) of racing for different inbreeding coefficients ($F_{ROH}$), wherein the left plot shows predictions alongside raw data (horses that have raced at 100% and those that have not raced at 0%), and the right plot zooms closer into the relevant plotting area;

**Figure 2** illustrates predicted probability (and 95% confidence intervals) of racing for different inbreeding coefficients $F_{ROH}$ based on (A) long ROH > 5 Mb and (B) short ROH < 5 Mb; wherein the left plots show predictions alongside raw data (horses that have raced at 100% and those that have not raced at 0%), and the right plots zoom closer into the relevant plotting area;

**Figure 3** illustrates GWAS of ROH effects on the probability of ever racing, wherein the Manhattan plot shows P-values for ROH effects on the probability of racing at every SNP location across the genome, and wherein, per SNP, two effects of ROH were tested to differentiate between ROH containing the minor allele and ROH containing the major allele, and wherein the hit on chromosome 14 corresponds to a negative effect of ROH on the probability of racing;

**Figure 4** illustrates distribution of inbreeding coefficients $F_{ROH}$ in thoroughbred horses;

**Figure 5** illustrates mean and standard deviation of inbreeding FROH in Thoroughbred cohorts born between 1996 and 2020 in Australia and New Zealand (N = 3,510) and Europe (N = 5,439);

**Figure 6** illustrates predicted probabilities of racing for AusNZ and EU from a model with an $F_{ROH} \times region$ interaction , wherein the slopes are virtually identical, indicating no difference in inbreeding effects between regions;

**Figure 7** illustrates region overview for the ~4Mb region surrounding the top GWAS hit (rs1144708552, 14:63883487 bp) showing the three protein-coding genes (FER, FBXL17, and EFNA5) in the region;

**Figure 8** illustrates FROH distribution among USA horses;

**Figure 9** illustrates a model based on Raced (0/1) ~ froh + sex + (1|birth_year);

**Figure 10** illustrates a model based on Raced (0/1) ~ FrohLong + FrohShort + sex + (1|birth_year);

**Figure 11** illustrates a model based on Races (> 0) ~ Froh + sex + (1|birth_year) + (1|olre), wherein Poisson model for number of races among horses that raced, wherein Olre is the observational level random effect account for overdispersion, and plots show the same prediction lines with and without raw data plotted on top;

**Figure 12** illustrates a model based on Races (> 0) ~ FrohLong + FrohShort + sex + (1|birth_year) + (1|olre);

**Figure 13** illustrates a GWAS model (Poisson, all ROH): races ~ sex + froh + snp_add + snp_roh1 + snp_roh2 + PC1-10 + (1|birth_year) + (1|olre), wherein the top hit was SNP rs69045681, Chr16, pos 8037334;

**Figure 14** illustrates ROH and SNP density at top hit;

**Figure 15** illustrates Manhattan plots (unsmoothed, top; smoothed, bottom) for a composite selections signals analysis comparing non-winners (TBO, male, n=59) to elite stallions (TBE, male, n=40), wherein Chromosome 1 contains 3 peaks, one of which includes the PYGL gene;

**Figure 16** illustrates rs1144708552 SNP genotype frequencies in cases and controls;

**Figure 17** illustrates proportion of cases with each genotype; and

**Figure 18** illustrates GWAS of long ROH (>5 Mb) effects on the probability of ever racing, wherein the Manhattan plot shows P-values for ROH effects on the probability of racing at every SNP location across the genome, wherein per SNP, two effects of ROH were tested to differentiate between ROH containing the minor allele and ROH containing the major allele.

## Examples

The present invention will now be described with reference to the appended non-limiting examples.

## Methods

Phenotypes, genotype filtering and imputation

SNP genotypes, race region and year of birth were available for $n$ = 8,951 thoroughbred horses. Race records (up to the end of the 2020 racing season) were retrieved for $n$ = 6,128 of these horses that were born in Europe (EUR) and Australia and New Zealand (ANZ) prior to and including 2015 and were therefore at least five years old. Among horses that race, the majority have their first start before they are five years old and the median age of retirement from racing has been five years old. Horses were assigned as 'raced' (n = 3,038, EUR, $n$ = 2,282 ANZ) if they had at least one start before five years old or 'unraced' (n = 606 EUR, $n$ = 202 ANZ) if they had no recorded race start before five years old. Race records for the major race regions (Europe, Australia and North America) were used to partition samples into the two cohorts searching all regions including other than birth region.

Two SNP genotyping platforms were used to genotype the animals; $n$ = 4,933 were genotyped on the Illumina Equine SNP70 BeadChip (Illumina, San Diego, CA) comprising approximately 70,000 SNPs (SNP70) and $n$ = 4,018 were genotyped on the Axiom Equine Genotyping Array (Axiom MNEC670) (Affymetrix, Santa Clara, CA) comprising approximately 670,000 SNPs (SNP670). All samples had a call rate >95%. Samples genotyped on the SNP70 array were imputed up to 488,576 SNPs with BEAGLE version 5.2 (Browning BL, Zhou Y, Browning SR. A One-Penny Imputed Genome from Next-Generation Reference Panels. Am J Hum Genet. 2018;103(3):338-48) using the samples genotyped on the SNP670 genotyping platform as a reference set (see Table 2 and Table 3 for full details of the analysis cohorts).

**Table 2: Sample sizes for analyses**

| Cohort | $N$ |
|---|---|
| Trends over time in EUR | 5,439 |
| Trends over time in ANZ | 3,512 |
| Effect of RoH on binary phenotype in EUR - raced vs unraced | 3,644 |
| Effect of RoH on binary phenotype in ANZ - raced vs unraced | 2,485 |

Table 3: Number and percent of horses that raced or were unraced.

|  | *n* EUR | *n* ANZ |
|---|---|---|
| **Raced** | 3,038 | 2,282 |
| U **nraced** | 606 | 202 |
| **% unraced** | 16.63 | 8.13 |

Post-imputation filtering and quality control

After imputation, SNPs with a Beagle dosage $R^2$ <0.8 were discarded to remove poorly imputed SNPs. SNPs with call rates >0.99, minor allele frequency ≥0.01 were then filtered for and only SNPs located on autosomes were retained, leaving a final dataset with 296,691 SNPs. Based on this dataset, we conducted a principal component analysis (PCA) in PLINK v1.90b (Purcell S., Neale B., Todd-Brown K., Thomas L., Ferreira M.A., Bender D., Maller J., Sklar P., de Bakker P.I., Daly M.J. & Sham P.C. (2007) PLINK: a tool set for whole-genome association and population-based linkage analyses. Am J Hum Genet 81, 559-75), which showed only minor population stratification between EUR and ANZ horses in the dataset.

Quantifying ROH and inbreeding ($F_{ROH}$)

ROH with a minimum length of 300 kb or 5 Mb were called using -homozyg in PLINK v1.90b with the following parameters: --homozyg-window-snp 30 -homozyg-snp 30 -homozyg-kb 300 -homozyg-gap 150 -homozyg-density 100 -homozyg-window-missing 1 -homozyg-window-het 1. 300 kb or 5 Mb was chosen as the minimum ROH length as interest was in evaluating fitness effects of both shorter and longer ROH. Moreover, based on the SNP density and horse genome size, it was expected around 40 SNPs in a 300 kb stretch of ROH, which should be sufficient to reliably call short ROH.

Inbreeding coefficients ($F_{ROH}$) were calculated by summing the total length of ROH for each individual and dividing by the autosomal genome length of 2,281 Mb (Wade CM, Giulotto E, Sigurdsson S, Zoli M, Gnerre S, Imsland F, et al. Genome sequence, comparative analysis, and population genetics of the domestic horse. Science. 2009;326(5954):865-7; McQuillan R, Leutenegger AL, Abdel-Rahman R, Franklin CS, Pericic M, Barac-Lauc L, et al. Runs of homozygosity in European populations. Am J Hum Genet. 2008;83(3):359-72). Shorter ROH with most-recent common ancestors from further back in the pedigree might differ in their fitness effects compared to long ROH. An inbreeding coefficient was therefore also calculated for ROH < 5 Mb ($F_{ROH\_short}$) and for ROH ≥ 5 Mb ($F_{ROH\_long}$). While the cutoff is semi-arbitrary, ROH of length 5 Mb are expected to have a common ancestor haplotype approximately 10 generations ago, calculated as 100/2 $\times$ g with g being the number of generations, assuming a uniform recombination map such that 1 Mb is equivalent to 1 cM. The effects of $F_{ROH\_long}$ and $F_{ROH\_short}$ can therefore be broadly interpreted as effects of more recent versus older inbreeding, or more precisely, of younger versus older haplotypes.

Modelling inbreeding effects on fitness to race

The effects of inbreeding on racing were modelled using general linear mixed models in lme4 (Bates DM, Mächler M, Bolker B, Walker S. Fitting linear mixed-effects models using lme4. Journal of Statistical Software 2015;67). A horse was assigned 0 if it had never raced and 1 if it had raced, and a binomial error distribution was used with logit link with the following model structure:

$$\Pr(raced_i = 1) = logit^{-1}(\beta_0 + F_{ROH_i}\beta_1 + region_i\beta_2 + sex_i\beta_3 + \alpha_k^{birth\ year})$$

$$\alpha_k^{birth\ year} \sim N(0, \sigma_{birth\ year}^2), \quad for\ k = 1, ..., 38$$

The probability of having raced $\Pr(raced_i = 1)$ was modeled with an intercept $\beta_0$ and fixed effects for individual inbreeding $F_{ROH}$, region (ANZ and EUR) and sex (F, M). Birth year was also fitted as a random effect in the model. The sample size was *n* = 6,128, with 2,484 and 3,644 horses from ANZ and EUR, respectively. To disentangle effects of short and long ROH, the model was refitted and $F_{ROH}$ was replaced with two predictors $F_{ROH\_short}$ and $F_{ROH\text{-}long}$.

Mapping loci underpinning inbreeding depression in fitness to race

To detect potential large effect loci involved in inbreeding depression and to distinguish these from additive SNP effects, a ROH-based genome-wide association study (GWAS) was performed (Pryce JE, Haile-Mariam M, Goddard ME, Hayes BJ. Identification of genomic regions associated with inbreeding depression in Holstein and Jersey dairy cattle. Genet Sel Evol. 2014;46:71; Stoffel MA, Johnston SE, Pilkington JG, Pemberton JM. Mutation load decreases with haplotype age in wild Soay sheep. Evol Lett. 2021 ;5(3):187-95). At every SNP location, a binomial mixed model was fitted with logit link using lme4 with the following structure:

$$\text{Pr}(raced_i = 1) = logit^{-1}(\beta_0 + SNP_{ROH_{allele.A_i}}\beta_1 + SNP_{ROH_{alleles_i}}\beta_2 + SNP_{ADD_i}\beta_3 + F_{ROH_{mod_i}}\beta_4$$

$$+ \; region_i\beta_5 + sex_i\beta_6 + \beta_{7-17}PC_{1-10} + \alpha_j^{birth\;year}$$

$$\alpha_k^{birth\;year} \sim N(0, \sigma_{birth\;year}^2), \quad for\;k = 1, \ldots, 38$$

The predictors of interest are $SNP_{ROH_{allelsAi}}$ and $SNP_{ROH_{allelsBi}}$, which are binary predictors quantifying whether an individual had a ROH overlapping a given SNP position with allele A as the homozygous genotype ($SNP_{ROH_{allelsAi}}$) or with allele B as the homozygous genotype ($SNP_{ROH_{allelsBi}} = 1$). When the SNP was not in a ROH it was coded as 0. These predictors indicate whether ROH have an effect on the probability of racing at a given position in the genome. The reason for fitting two ROH predictors is that a large effect deleterious allele is likely to occur only on certain haplotypic backgrounds. To ensure that a ROH effect is not simply an additive effect, a predictor with the SNP genotype coded as 0,1,2 for homozygous, heterozygous, and homozygous for the alternative allele was also fitted. $F_{ROH_{mod}}$ is the individual inbreeding coefficient calculated from all autosomes except for the chromosome containing the focal SNP. This ensures that the estimated effects are local ROH effects separate to the genome-wide inbreeding level. As before, region and sex were fitted as further fixed effects as well as ten principal components based on the variance-standardized additive relationship matrix to account for relatedness within the sample. Lastly, year of birth was included as a random effect. For each model, the model estimates for both ROH effects and their P-values calculated using Wald-Z tests were extracted. A genome-wide significance threshold was determined by calculating the effective number of tests when accounting for linkage disequilibrium using 'simpleM' (Gao X, Starmer J, Martin ER. A multiple testing correction method for genetic association studies using correlated single nucleotide polymorphisms. Genet Epidemiol. 2008;32(4):361-9). This led to an estimated 90,900 independent tests, which we doubled (as two tests per model were performed), before using this value for a Bonferroni correction of P-values, leading to a genome-wide significance threshold of $P < 2.75 \times 10^{-7}$.

Candidate gene region selection

The top-ranking 1% of SNPs in the four GWAS were investigated to identify candidate regions for further investigation. Regions containing top ranking SNPs, clusters of >1 SNP among the highest ranking SNPs, and SNPs identified in more than one GWAS were prioritised.

Variant identification in thoroughbred whole-genome sequence data

A catalogue of genetic variants and predicted functional consequences was derived from 30 thoroughbred whole-genome sequences. The functional consequences of the variants (including SIFT scores (Ng P.C. & Henikoff S. (2003) SIFT: Predicting amino acid changes that affect protein function. Nucleic Acids Res 31, 3812-4) for missense variants were predicted with the Ensembl Variant Effect Predictor (VEP; McLaren W., Gil L., Hunt S.E., Riat H.S., Ritchie G.R., Thormann A., Flicek P. & Cunningham F. (2016) The Ensembl Variant Effect Predictor. Genome Biol 17, 122; (version 91.3). These data formed the reference files that were screened for putative causative / functional variants in regions/genes of interest. Prioritisation of variants was performed using previously described methods ( Jalali Sefid Dashti M. & Gamieldien J. (2017) A practical guide to filtering and prioritizing genetic variants. Biotechniques 62, 18-30) with a particular focus on (a) predicted effect of the variant, where 'high' effect was selected where possible, (b) biological function of the gene relevant to the disease, (c) minor allele frequency $\geq 0.1$, and (d) high quality score (most had QUAL=999). Variants were only considered if they were in known genes. Predicted 'high' effect variants included the annotations splice_acceptor_variant&intron_variant, frameshift_variant, splice_acceptor_variant, and stop_gained, and predicted 'moderate' effect variants included the annotations missense_variant and inframe_deletion.

**Example 1**

Patterns of inbreeding in the genome and across time

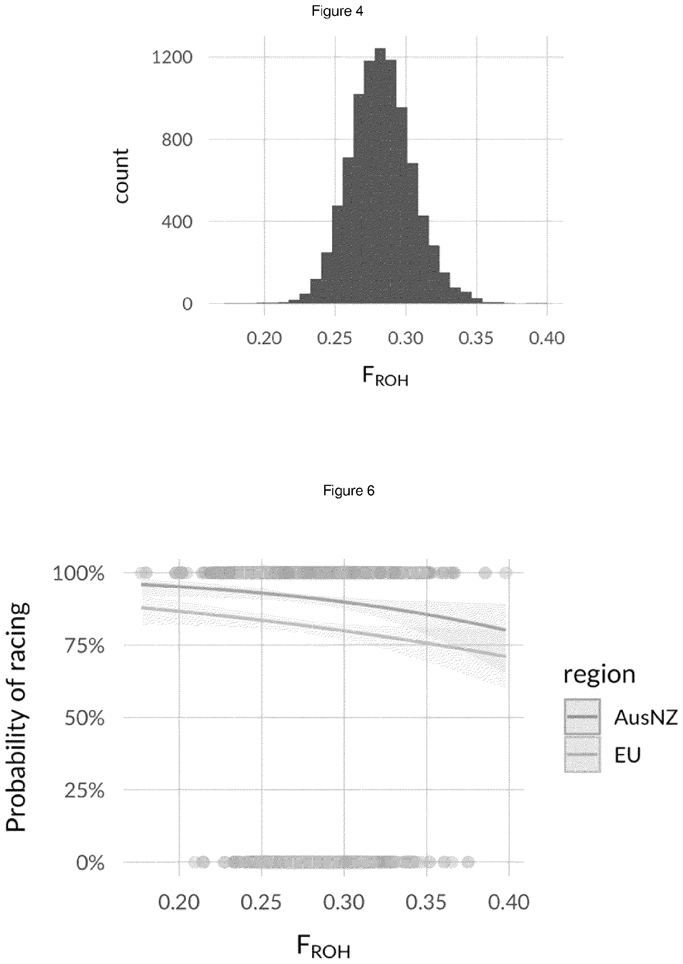

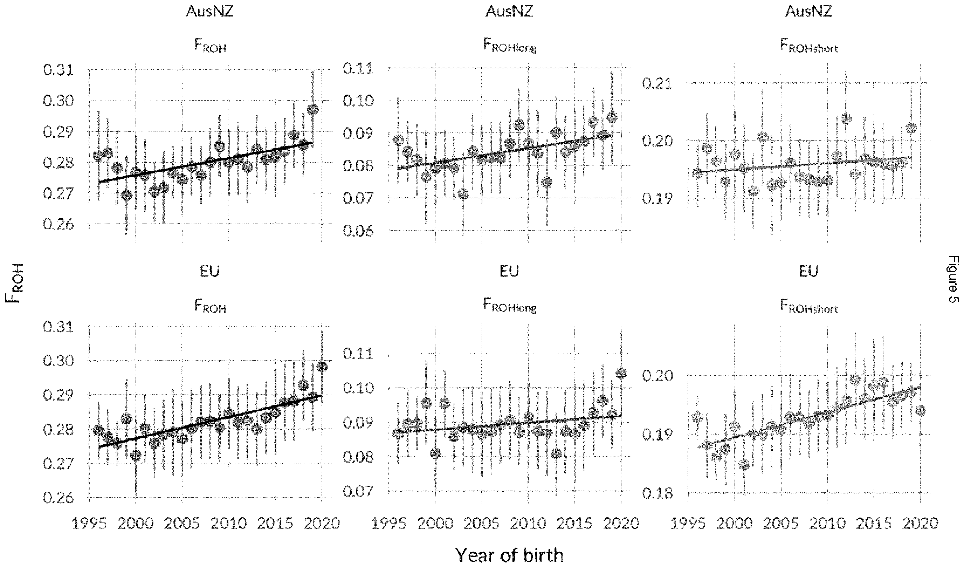

ROH were evaluated using 297K SNP genotypes generated for $n$ = 8,951 thoroughbred horses born in Europe (EUR) and Australia/New Zealand (ANZ) between 1971 and 2020. On average, individual animals had 415 (range 229-994) ROH segments >300 kb with a mean length of 1.5 Mb (the longest ROH spanned 67 Mb) that covered -28% of each genome (mean $F_{ROH}$ = 0.28; range 0.18-0.40) (see Figure 4). An increase in $F_{ROH}$ in both EUR and ANZ over time was observed (see Figure 5). In ANZ, the increase in inbreeding appears to be mostly driven by long ROH segments (≥5 Mb) but in EUR it is driven largely by short ROH segments (<5 Mb) (see Figure 5).

**Example 2**

Inbreeding effects on racing

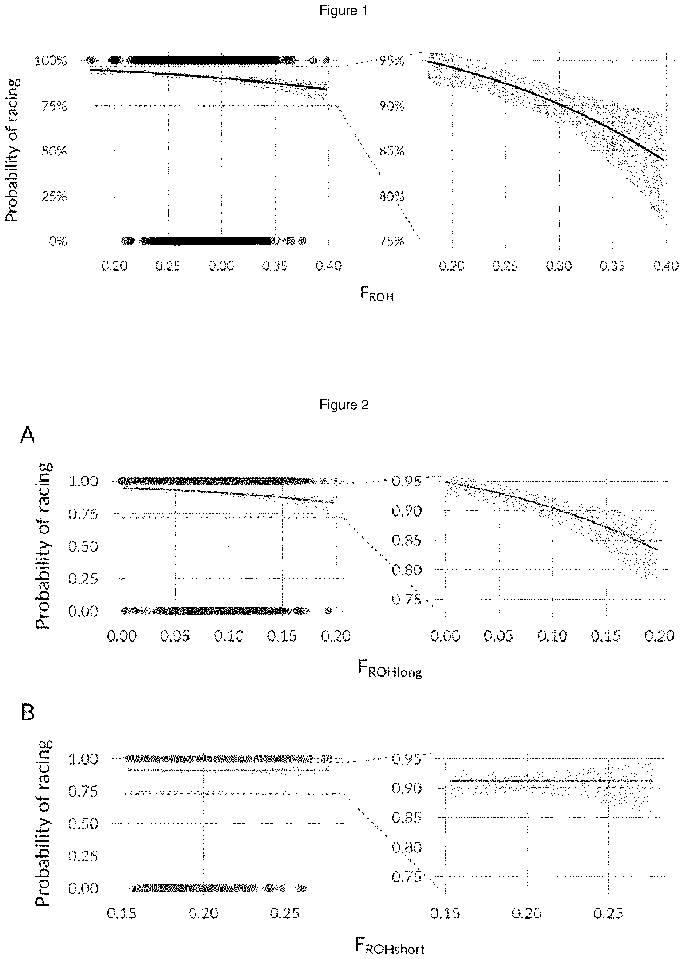

To test the hypothesis that inbreeding influences failure to race, the effects of inbreeding ($F_{ROH}$) on the probability of racing among $n$ = 6,128 horses was modelled using general linear mixed models. The log-odds of racing decreased significantly ($P$ = 0.0009) with increasing $F_{ROH}$ (log odds-ratio (log(OR) [95% confidence interval (CI) = -5.75 [-9.15, -2.36], see Table 4), corresponding to a predicted decrease in the odds of racing by 44% for a 10% increase in $F_{ROH}$. It is also possible to translate these effects into predicted probabilities of racing (see Figure 1). According to the model, a horse with the highest observed inbreeding coefficient ($F_{ROH}$ = 0.40) had roughly a 13% lower probability of ever racing than a horse with the lowest observed inbreeding coefficient ($F_{ROH}$ = 0.18). Horses that were 10% more inbred than average had a 7% lower probability of ever racing, while horses with a $F_{ROH}$ 10% lower than the mean had a 4% higher probability of racing (see Table 5 for numeric predictions). A model was also fitted with an interaction between $F_{ROH}$ and region (EUR, ANZ) to quantify a potential difference in inbreeding depression between regions, but the slopes were very similar and the interaction was not significant (see Figure 6, Table 6).

**Table 4:** Model estimates for a binomial mixed model with the response variable 'raced'(0/1 for whether a horse has ever raced), wherein the fixed effect estimates are on the log-odds scale, the estimate for FROH can be interpreted as the difference in the log-odds of racing between a completely outbred (FROH = 0) and a completely inbred horse (FROH=1), and dividing the estimate by 10 gives the difference in log-odds for a 10% increase in FROH and exponentiating the estimate gives the change in the odds of racing.

| term | estimate (log-OR) | CI (2.5%) | CI (97.5%) | z-value | p-value | Info |
|---|---|---|---|---|---|---|
|  |  |  |  |  |  |  |
| Intercept | 3.941 | 2.959 | 4.923 | 7.867 | 0 |  |
| Fixed effects |  |  |  |  |  |  |
| $F_{ROH}$ | -5.753 | -9.151 | -2.355 | -3.318 | 0.001 | continuous |
| Region | -0.854 | -1.032 | -0.676 | -9.4 | 0 | categorical (0=AusNZ, 1=EU) |
| term | estimate (log-OR) | CI (2.5%) | CI (97.5%) | z-value | p-value | Info |
| Sex | 0.48 | 0.324 | 0.637 | 6.012 | 0 | categorical (0=female, 1=male) |
| Random effects (standard deviation) |  |  |  |  |  |  |
| Birth year | 0.3 | NA | NA | NA | NA | n = 38 |

**Table 5:** Predicted probability of racing for horses with different inbreeding coefficients $F_{ROH}$ relative to the average inbreeding coefficient.

| $F_{ROH}$ | Relative | Probability of racing | CI (2.5%) | CI (97.5%) |
|---|---|---|---|---|
| 0.18 | -10% | 94.8% | 92.4% | 96.5% |
| 0.23 | -5% | 93.2% | 91.2% | 94.8% |
| 0.28 | mean | 91.1% | 89.2% | 92.8% |
| 0.33 | +5% | 88.5% | 85.4% | 91% |
| 0.38 | +10% | 85.3% | 79.5% | 89.6% |

**Table 6:** Model estimates for a binomial mixed model with the response variable 'raced' (0/1 for whether a horse has ever raced) and an interaction between $F_{ROH}$ and Region.

| term | estimate (log-OR) | CI (2.5%) | CI (97.5%) | z-value | p-value | Info |
|---|---|---|---|---|---|---|
| | | | | | | |
| Intercept | 4.58 | 2.724 | 6.435 | 4.837 | 0 | |
| Fixed effects | | | | | | |
| $F_{ROH}$ | -8.002 | -14.498 | -1.507 | -2.415 | 0.016 | continuous |
| Region | -1.727 | -3.896 | 0.441 | -1.561 | 0.118 | categorical (0=AusNZ, 1=EU) |
| Sex | 0.48 | 0.323 | 0.636 | 6.003 | 0 | categorical (0=female, 1=male) |
| $F_{ROH}$ * Region | 3.086 | -4.538 | 10.709 | 0.793 | 0.428 | interaction |
| Random effects (standard deviation) | | | | | | |
| Birth year | 0.302 | NA | NA | NA | NA | n = 38 |

**Example 3**

Inbreeding effects on racing by ROH length

Long ROH had a strong negative effect on probability of racing, while short ROH had no effect (see Figure 2, Table 7), indicating that recent inbreeding rather than historic inbreeding is the cause of inbreeding depression for this trait in the population. The predicted log-odds of racing decreased with increasing $F_{ROH\_long}$ (log(OR) [95% CI] = -6.57 [-10.04, -3.11]) corresponding to a predicted decrease in the odds of racing by 48% for a 10% increase in $F_{ROH\_long}$.

**Table 7:** Model estimates for a binomial mixed model with the response variable 'raced' (0/1 for whether a horse has ever raced) for $F_{ROHlong}$ and $F_{ROHshort}$, wherein the fixed effect estimates are on the log-odds scale. Individual inbreeding $F_{ROH}$ has been split into two predictors, representing the proportion of the genome in long ROH > 5Mb ($F_{ROHlong}$) and shorter than 5Mb ($F_{ROHshort}$).

| term | estimate (log-OR) | CI (2.5%) | CI (97.5%) | z-value | p-value | Info |
|---|---|---|---|---|---|---|
| | | | | | | |
| Intercept | 2.908 | 1.588 | 4.227 | 4.319 | 0 | |
| Fixed effects | | | | | | |
| $F_{ROHlong}$ | -6.575 | -10.036 | -3.113 | -3.723 | 0 | continuous |
| $F_{ROHshort}$ | -0.02 | -5.993 | 5.953 | -0.007 | 0.995 | continuous |

(continued)

| Fixed effects | | | | | | |
|---|---|---|---|---|---|---|
| Region | 0.478 | 0.321 | 0.634 | 5.981 | 0 | categorical (0=AusNZ, 1=EU) |
| Sex | -0.843 | -1.021 | -0.664 | -9.251 | 0 | categorical (0=female, 1=male) |
| Random effects (standard deviation) | | | | | | |
| Birth year | 0.309 | NA | NA | NA | NA | n = 38 |

**Example 4**

**Identification of a large-effect haplotype**

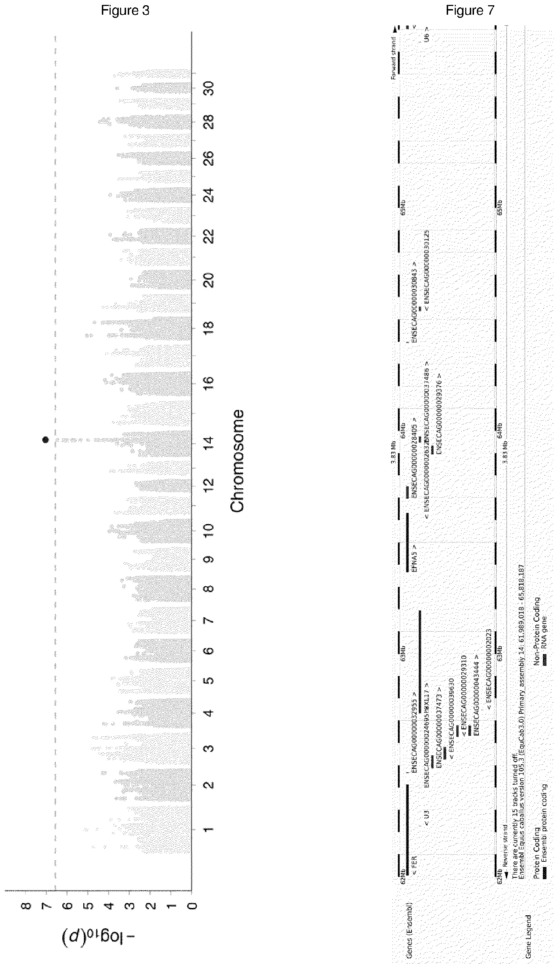

The GWAS uncovered a single significant hit ($P$ = 9.09 $\times$ 10^{-8}) for a ROH overlapping the minor allele A at SNP rs1144708552 at position 63883487 bp on ECA14 (log(OR) [95% CI] = -1.82 [-2.48, -1.15], see Figure 3, Table 8). While only 64 individuals (1%) were homozygous for the A allele and had overlapping ROH (ROH+AA), these horses had a 32% lower predicted probability of ever racing (see Table 9) when keeping other factors such as $F_{ROH}$ constant. The 64 ROH+AA horses were the progeny of 40 sires. 31.25% of ROH+AA horses were unraced, compared to the sample average 13.1%. Furthermore, ROH+AA horses that did race had on average 13.3 races, compared to the sample average of 16.3 races, suggesting that while some ROH+AA horses may race, they are less durable. Notably, genome-wide inbreeding ($F_{ROH}$) also showed a strong effect on probability of racing in the same model (log(OR) [95% CI] = -6.39 [-9.88, -2.90], see Table 8). Consequently, inbreeding depression in the thoroughbred is largely due to the genome-wide effects of many recessive deleterious mutations in addition to a larger effect recessive mutation expected to be in proximity to SNP rs1144708552.

Table 8: Model estimates for the GWAS model at the top hit, wherein ROH [minor allele, A] is the hit, indicating that horses with an ROH containing two copies of the minor allele (A) have lower odds of ever racing, SNP (additive) is the additive SNP effect which is usually the factor of interest in GWAS, but has been added here to ensure that the estimated ROH effect is not simply an additive SNP effect, and wherein individual inbreeding $F_{ROH}$ is the ROH based inbreeding coefficient at all chromosomes except for chromosome 14 which contains the focal region.

| term | estimate (log-OR) | CI (2.5%) | CI (97.5%) | z-value | p-value | Info |
|---|---|---|---|---|---|---|
| | | | | | | |
| Intercept | 4.769 | 3.671 | 5.868 | 8.513 | 1.70 $\times$ 10^{-17} | |
| Fixed effects | | | | | | |
| ROH [minor allele, A] | -1.815 | -2.48 | -1.149 | -5.344 | 9.09 $\times$ 10^{-8} | categorical (0=no ROH, 1=ROH) |
| ROH [major allele, G] | 0.066 | -0.133 | 0.264 | 0.649 | 5.16 $\times$ 10^{-1} | categorical (0=no ROH, 1=ROH) |
| SNP (additive) | -0.333 | -0.543 | -0.123 | -3.111 | 1.86 $\times$ 10^{-3} | continuous (hom(min)=0, het=1,hom(m aj)=2) |
| $F_{ROH}$ | -6.393 | -9.882 | -2.904 | -3.592 | 3.29 $\times$ 10^{-4} | continuous (0-1) |
| Region | -0.89 | -1.125 | -0.654 | -7.412 | 1.24 $\times$ 10^{-13} | categorical (0=AusNZ, 1=EU) |
| Sex | 0.47 | 0.313 | 0.628 | 5.85 | 4.92 $\times$ 10^{-9} | categorical (0=female, 1=male) |
| PC1 | -6.358 | -14.274 | 1.559 | -1.574 | 1.15 $\times$ 10^{-1} | continuous |

(continued)

| Fixed effects | | | | | | |
|---|---|---|---|---|---|---|
| PC2 | -3.916 | -11.274 | 3.442 | -1.043 | $2.97 \times 10^{-1}$ | continuous |
| PC3 | 4.379 | -2.458 | 11.216 | 1.255 | $2.09 \times 10^{-1}$ | continuous |
| PC4 | 6.017 | -2.238 | 14.273 | 1.429 | $1.53 \times 10^{-1}$ | continuous |
| PC5 | -1.724 | -9.819 | 6.371 | -0.417 | $6.76 \times 10^{-1}$ | continuous |
| PC6 | -5.057 | -12.641 | 2.527 | -1.307 | $1.91 \times 10^{-1}$ | continuous |
| PC7 | 9.382 | 2.267 | 16.497 | 2.584 | $9.76 \times 10^{-3}$ | continuous |
| PC8 | 9.395 | 2.25 | 16.54 | 2.577 | $9.96 \times 10^{-3}$ | continuous |
| PC9 | -4.979 | -12.275 | 2.318 | -1.337 | $1.81 \times 10^{-1}$ | continuous |
| PC10 | 9.223 | 1.617 | 16.829 | 2.377 | $1.75 \times 10^{-2}$ | continuous |
| Random effect (standard deviation) | | | | | | |
| Birth year | 0.286 | NA | NA | NA | NA | n = 38 |

**Table 9:** Predicted probabilities of racing for horses with an ROH and minor allele at the top hit (1) vs. without (0), wherein predictions were calculated using marginal means by keeping region and sex in the model constant at their mean, and wherein individual inbreeding $F_{ROH}$ was kept constant at the mean to estimate the predicted difference in racing for individuals with average inbreeding coefficients.

| ROH [minor allele, A] | Probability of racing | CI (2.5%) | CI (97.5%) |
|---|---|---|---|
| 0 | 0.890 | 0.873 | 0.905 |
| 1 | 0.569 | 0.403 | 0.721 |

Using phased genotype data, haplotypes surrounding the rs1144708552 SNP were screened. Specifically, haplotypes of length 1 Mb, including all SNPs occurring up to 500 kb on each side of rs1144708552 were the focus. Among ROH+AA horses, 78.1% were homozygous for an identical haplotype (see Table 10), which we refer to as THR14. All but one ROH+AA horse carried at least one exact copy of THR14, and where haplotypes differed from THR14, this was on average by only 2 out of 84 base positions that make up the haplotype. THR14 was significantly (*P* = 0.006) overrepresented among breeding stallions (*n* = 130), where it had a frequency of 15.4% compared to 10.1% in the overall sample.

**Table 10:** Haplotype structure around the top GWAS hit, SNP rs1144708552, wherein a haplotype size of 500 Kb in both directions from SNP rs114470855 was chosen, which included 84 SNPs in total, wherein the table shows all haplotypes with frequencies > 0.1% in this sample, and the highlighted row is the THR14 haplotype containing the putative deleterious recessive variant, wherein N and % specify the count and frequency of each haplotype in the population, respectively.

| Haplotype | N | % |
|---|---|---|
| TGGACCGAGACAAACAGATTGGTTCTAATATGAGTCGGGTTCGCAGACAGATTGT GAGCAAAGGGACATGCTCTCCTTTGGACT | 3610 | 29.45 |
| TGAACCGAGACAAACAGATTGGCCTTAATATAAACTGGGTCAGCAGACAGATTGT GAACAAAGGGGTACATCATTCTTTGGACT | 2305 | 18.81 |
| CGGGCCGGGAAAGACAAACCTGCCTTAATATAAACTAGGTCCATGGGCGAATTAT AGGAGAAGGGACGTGCTCTCCTTTGGACT | 1234 | 10.07 |
| CGGACCGAGACAAAAAGATTGGCCTCGGCAGACGTCGTGCCCACAGACAAGTTA TGAGAAAGAGAACACACCATTTCCGGAACT | 980 | 8.00 |
| TGGACCAAGACAAACAGATTGGTTCTGGCAGAAGCCGGGTCCATAGATAAACCGG GGGAGGAAAGACACACCATCCTTTTGACT | 925 | 7.55 |
| TGGAATGATGAGGGCCGGCCTGTTCTGGTGTACGTCGGATCCATAAACGGACCG GGAGAAAGAGAACACACCATTTCCGGAACT | 787 | 6.42 |
| TAGAATGATGAGGGCCGGCCTATTCTAATATGAGTCGGATCAGCAGACAGATTGT AGGAGAAGGGACGTGCTCTCTTCTGGACT | 452 | 3.69 |
| TGGACCGAGACAAAAAGATTGGCCTCGGCAGACGTCGGGTCCATAGATAAACCG GGGGAGGAAAGACATGTTAGTTCCTGACTC | 156 | 1.27 |
| CGGGCCGGGAAAGACAAACCTATTCTAATATGAGTCGGATCAGCAGACAGATTGT AGGAGAAGGGACGCACCATTTCCGGAACT | 128 | 1.04 |

EP 4 286 534 A1

**Example 5**

Candidate genes for survival / viability

Since most (-80%) of the ROH in the identified region extend across >2.8 Mb, a 3 Mb region surrounding the rs1144708552 SNP (+/-1.5 Mb) was searched for genes with biological functions that may impact on survival or viability of a horse. The most compelling candidate gene among the three protein-coding genes (*FER, FBXL17, EFNA5*) in the region was *EFNA5* (ECA14: 63365481-63631761 bp), which was also the closest (-250 kb) gene to the rs1144708552 SNP (see Figure 7).

**Example 6**

**THR14 haplotype, rs1144708552 and musculoskeletal injury risk**

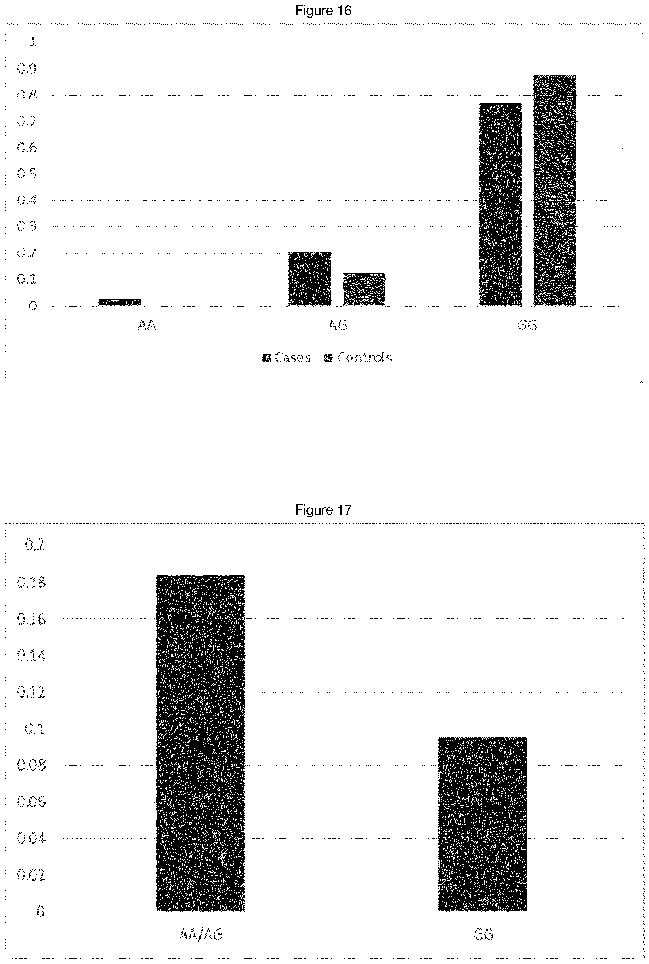

Due to its known biological function, it was hypothesised that homozygosity at the EFNA5 locus may be associated with breakdown injury in thoroughbreds. Musculoskeletal injury phenotypes and rs1144708552 SNP genotypes were available for n=364 (n=39 cases, n=325 controls) horses-in-training with the same trainer (year of birth 2007-2014), with only one individual (case, broken pelvis) homozygous for the minor allele (A). The GG genotype was suggestive of a protective effect (chi-square = 3.47, P=0.063) with 23% of cases either AA or AG and 12.3% of controls AG (see Figure 16). Horses with the AA or AG genotype were 1.9 times more likely to have sustained an injury (18.4%) than GG genotype (9.5%) horses (Figure 17). These results are suggestive of a relationship between the EFNA5 locus and injury risk.

**Example 7**

**Analyses using horses born and raced in USA**

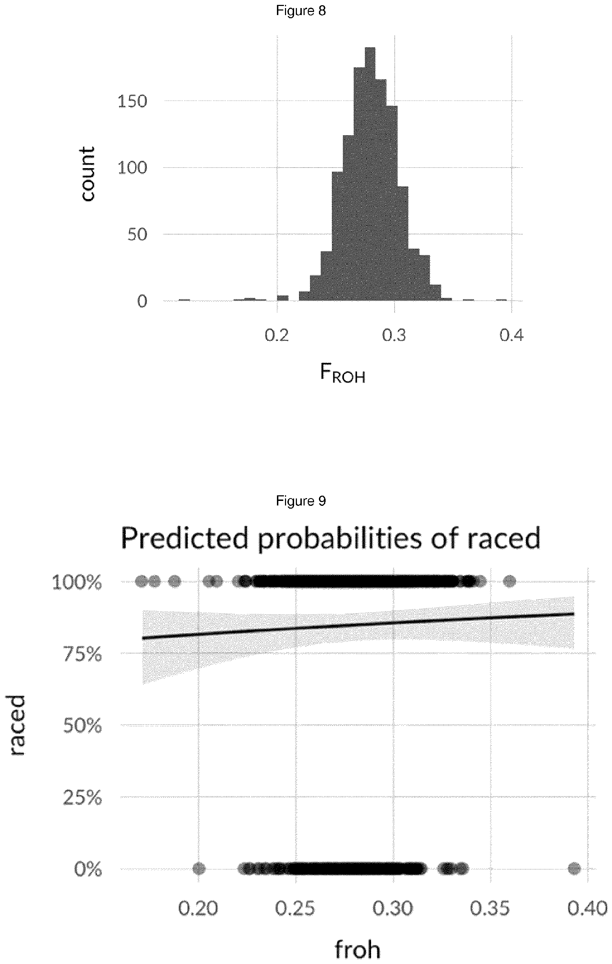

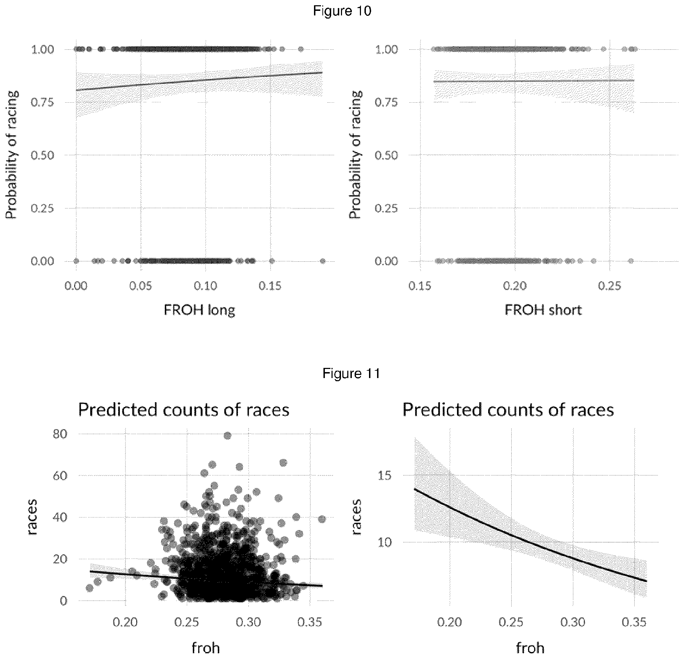

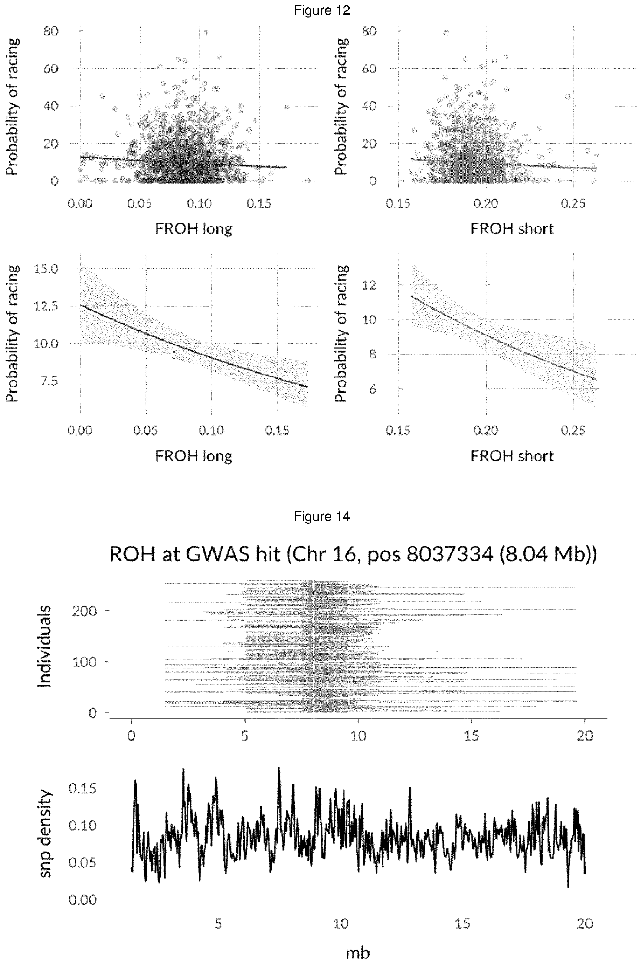

Inbreeding was quantified based on ROH using 297K SNP genotypes from 1,136 horses born in USA, of which 17.3% were unraced. The distribution of $F_{ROH}$ was similar to the EU/ANZ distribution (see Figure Z). In USA, $F_{ROH}$ is not associated (P=0.394) with the probability of ever racing (see Figure 9, Table 11); separating ROH into long and short ROH also showed no effect on probability of ever racing (Figure 10, Table 12). However, $F_{ROH}$ is significantly associated (P=0.001) with durability (number of race starts) among horses that do race (n=940) (Figure 11, Table 13). Long ROH (> 5Mb) and short ROH have similar effects on the number of race starts (Figure 12, Table 14). Overall, horses with a 10% higher $F_{ROH}$ than the average inbreeding coefficient were predicted to have 3.5 fewer starts compared to horses with an average inbreeding coefficient (Table 15). The model predicts approximately 17 race starts for horses with the lowest $F_{ROH}$ (0.17), but only 8 starts for horses with the highest $F_{ROH}$ (0.39).

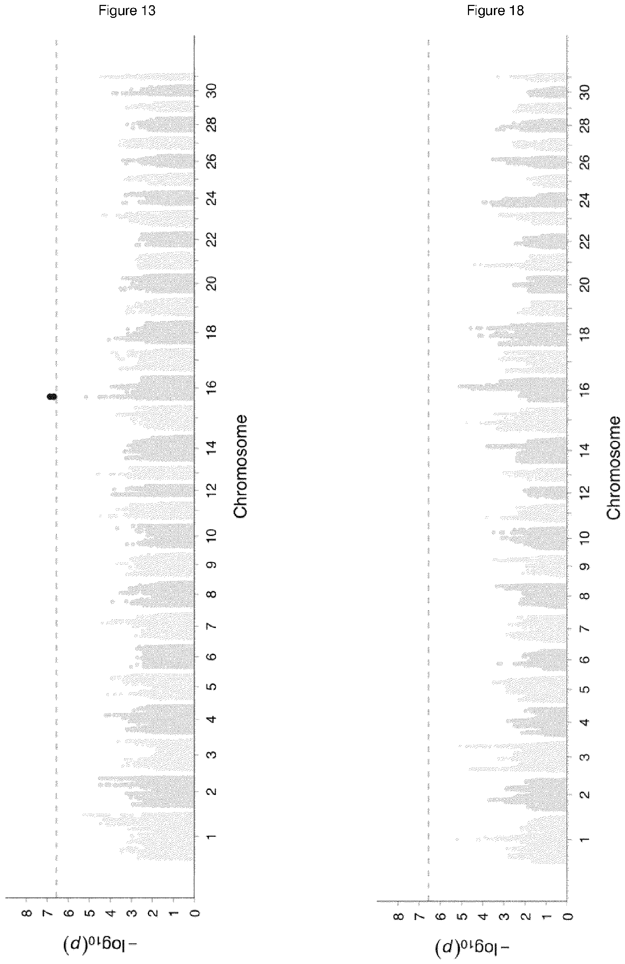

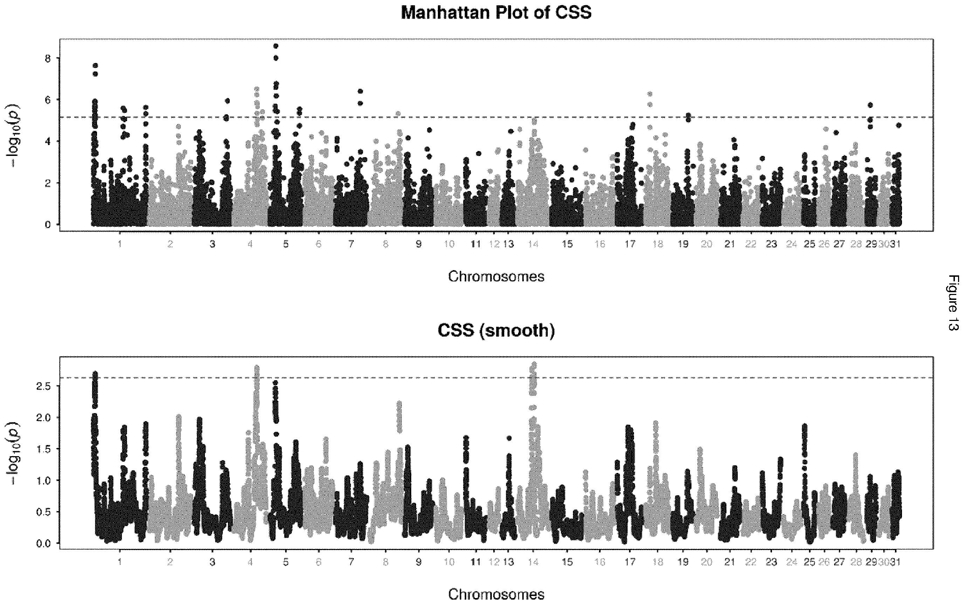

A ROH-based genome-wide association study (GWAS) identified a significant peak on ECA16 containing two SNPs within 7305 bp of each other (Figure 13). The top hit was rs69045681 located on chromosome 16 at position 8037334 bp. In its homozygous state, the haplotype on ECA16 containing the minor allele A, is predicted to result in six fewer race starts (4.8) than the predicted number of starts for other horses (11.1) (Table 16). Thirty individuals had an ROH containing the AA genotype, *i.e.* 3.2 % of the individuals in the GWAS dataset (n = 940), which include only USA individuals that raced at least once. The ROH and SNP densities at the top hit show relatively even distributions (Figure 14).

The average length of ROH in the region overlapping the top hit was 5178 kb. Therefore, we searched for genes within the 5 Mb region surrounding the top SNP (*i.e.* 2.5 Mb up and down stream). The gene closest to the top SNP is a non-coding RNA, eca-mir-885 (ENSECAG00000026204). In humans and other mammalian species, miR-885 functions in a range of biological systems including in muscle, where it promotes proliferation of myoblasts and is involved in the determination of fibre type; in the heart, where it protects against hypoxia and promotes right ventricular hypertrophy; and in bone, where it is associated with fractures in osteosarcoma. In the horse, eca-mir-885 is among the six downregulated genes in venous blood samples collected from horses diagnosed with subclinical traumatic primary tibiotarsal osteoarthritis compared to controls. Therefore, this haplotype may contribute to traits relating to bone dysfunction in thoroughbred.

The third ranked SNP in the GWAS ((P= $5.23 \times 10^{-06}$) was rs1143204553, located on ECA1 at position 186974288bp. While the SNP did not reach the threshold for genome-wide significance, 27 (90%) of the top ranked 0.0001% of SNPs (i.e. 30 SNPs) in the GWAS were within a 1.6 Mb region (Table 18). The region was approximately centred at 18.6 Mb, the location of the *PYGL* gene (186995497-187024683 bp). *PYGL* encodes glycogen phosphorylase L, and in humans a mutation in the gene is associated with glycogen storage disease.

*PYGL* has been identifies within a region defined by the top 1% SNPs under selection among poor-performing horses in a composite-selection signals analysis comparing elite (n=40) stallions to non-elite (n=59) male horses. 46,478 autosomal SNPs (chr 1-31; MAF > 0.01, missing genotyping rate <0.05) were included. N = 40 elite stallions and n=59 non-elite males were included following removal of closely related individuals (pi-hat=0.25). Missing genotypes were imputed and haplotypes phased using BEAGLE. Each test - Fst, SAF (selected allele frequency), and XP-EHH (across populating extended haplotype homozygosity) was ranked from the fractional ranks to mean Z-values and P-value. The composite selection signature is a weighted index calculated from the different test estimate. Results were smoothed from single SNP based CSS to window-based CSS (1Mb) to reduce spurious signals (Figure 15). The top 0.1% of smoothed CSS scores are defined as a significant SNP. Significant genomic regions were identified by including regions containing five SNPs among the top 1% of SNPs (Table 19). Genes were mined in the regions ±0.5Mb from the flanking SNPs. The 11th ranked region contained PYGL. Also among the selected regions was the region on chromosome 14 that contains the THR14 haplotype and the EFNA5 gene.

Glycogen phosphorylase catalyses the rate-limiting step in glycogenolysis in animals by releasing glucose-1-phosphate from the terminal alpha-1,4-glycosidic bond. In some breeds of horse glycogen storage disease is known as Equine polysaccharide storage myopathy (EPSM). This is a form of rhabdomyolysis classified as a metabolic disease that results in the accumulation of high muscle glycogen and abnormal polysaccharide in skeletal muscles. Recurrent exertional rhabdomyolysis (RER, 'tying up') is a common disease in thoroughbred horses. Exertional rhabdomyolysis (ER) is a group of muscle disorders associated with exercise observed in athletic horses, with clinical characteristics of muscle stiffness, cramping, pain and exercise intolerance accompanied by elevations in the plasma muscle-derived enzymes creatine kinase (CK) and aspartate aminotransferase (AST). ER can be subdivided into traumatic (e.g. strains, tears), sporadic/acute (e.g. lack of fitness) and inherited causes. RER is an inherited chronic exertional myopathy with an estimated heritability in thoroughbreds between 0.34-0.46, although to-date, genetic mutations associated with RER susceptibility have not been described. RER occurs in approximately 5-10% of racing thoroughbreds and is diagnosed based on clinical signs and elevations in plasma CK and AST. Numerous studies have identified stress as a major risk factor for development of clinical episodes of RER, occurring most frequently in young, nervous females. Since prevention of further episodes of RER in susceptible horses primarily involves regular exercise and minimizing stress, identifying horses with reduced ability to cope with stress before they enter a training environment would allow proactive management of this disease.

**Table 11: Model 1: Raced (0/1) ~ froh + sex + (1|birth_year)**

| term | estimate (log-OR) | CI (2.5%) | CI (97.5%) | z-value | p-value | Info |
|---|---|---|---|---|---|---|
| | | | | | | |
| Intercept | 0.891 | -1.034 | 2.817 | 0.907 | 0.364 | |
| Fixed effects | | | | | | |
| $F_{ROH}$ | 2.959 | -3.838 | 9.756 | 0.853 | 0.394 | continuous |
| Sex | -0.068 | -0.41 | 0.273 | -0.392 | 0.695 | categorical (0=female, 1=male) |
| Random effects (standard deviation) | | | | | | |
| Birth year | 0.489 | NA | NA | NA | NA | n = 9 |

**Table 12: Model 2: Raced (0/1) ~ FrohLong + FrohShort + sex + (1|birth_year)**

| term | estimate (log-OR) | CI (2.5%) | CI (97.5%) | z-value | p-value | Info |
|---|---|---|---|---|---|---|
| | | | | | | |
| Intercept | 1.342 | -1.243 | 3.927 | 1.018 | 0.309 | |
| Fixed effects | | | | | | |
| $F_{ROHlong}$ | 3.451 | -3.589 | 10.492 | 0.961 | 0.337 | continuous |
| $F_{ROHshort}$ | 0.409 | -11.487 | 12.305 | 0.067 | 0.946 | continuous |
| Sex | -0.07 | -0.414 | 0.275 | -0.396 | 0.692 | categorical (0=female, 1=male) |
| Random effects (standard deviation) | | | | | | |
| Birth year | 0.487 | NA | NA | NA | NA | n = 9 |

**Table 13: Model 3: Races (> 0) ~ Froh + sex + (1 |birth_year) + (1|olre).** Note: Poisson model for number of races among horses that raced. Olre is the observational level random effect account for overdispersion. Plots show the same prediction lines with and without raw data plotted on top.

| term | estimate (log-OR) | CI (2.5%) | CI (97.5%) | z-value | p-value | Info |
|---|---|---|---|---|---|---|
| | | | | | | |
| Intercept | 3.256 | 2.65 | 3.862 | 10.529 | 0 | |
| Fixed effects | | | | | | |
| $F_{ROH}$ | -3.619 | -5.769 | -1.468 | -3.298 | 0.001 | continuous |
| Sex | 0.42 | 0.309 | 0.531 | 7.421 | 0 | categorical (0=female, 1=male) |
| Random effects (standard deviation) | | | | | | |
| Birth year | 0.699 | NA | NA | NA | NA | n = 9 |
| Observation-level random effect | 0.114 | NA | NA | NA | NA | n = 940 |

**Table 14: Model 3: Races (> 0) ~ FrohLong + FrohShort + sex + (1|birth_year) + (1|olre)**

| term | estimate (log-OR) | CI (2.5%) | CI (97.5%) | z-value | p-value | Info |
|---|---|---|---|---|---|---|
| | | | | | | |
| Intercept | 3.537 | 2.711 | 4.363 | 8.39 | 0 | |
| Fixed effects | | | | | | |
| $F_{ROHlong}$ | -3.312 | -5.548 | -1.076 | -2.904 | 0.004 | continuous |
| $F_{ROHshort}$ | -5.208 | -9.049 | -1.368 | -2.658 | 0.008 | continuous |
| Sex | 0.42 | 0.309 | 0.53 | 7.412 | 0 | categorical (0=female, 1=male) |
| Random effects (standard deviation) | | | | | | |
| olre | 0.699 | NA | NA | NA | NA | n = 940 |
| Birth year | 0.111 | NA | NA | NA | NA | n = 9 |

**Table 15: Predicted number of races for horses with varying inbreeding coefficients $F_{ROH}$**

| $F_{ROH}$ | Relative | Predicted number of races | CI (2.5%) | CI (97.5%) |
|---|---|---|---|---|
| 0.18 | -10% | 16.691 | 13.197 | 21.11 |
| 0.23 | -5% | 13.928 | 12.067 | 16.077 |
| 0.28 | mean | 11.623 | 10.564 | 12.788 |
| 0.33 | +5% | 9.699 | 8.397 | 11.203 |
| 0.38 | +10% | 8.094 | 6.394 | 10.245 |

**Table 16: Predicted number of races for ROH + minor allele at top hit.**

| ROH [minor allele, A] | Predicted number of races | CI (2.5%) | CI (97.5%) |
|---|---|---|---|
| 0 | 11.118 | 10.188 | 12.132 |
| 1 | 4.804 | 3.506 | 6.581 |

**Table 17: Full model at top GWAS hit**

| term | estimate (log-OR) | CI (2.5%) | CI (97.5%) | z-value | p-value | Info |
|---|---|---|---|---|---|---|
| Intercept | 3.172 | 2.552 | 3.792 | 10.03 | $1.13 \times 10^{-23}$ | |
| Fixed effects | | | | | | |
| ROH [minor allele, A] | -0.839 | -1.151 | -0.528 | -5.28 | $1.29 \times 10^{-7}$ | categorical (0=no ROH, 1=ROH) |
| ROH [major allele, G] | -0.045 | -0.191 | 0.101 | -0.605 | $5.45 \times 10^{-1}$ | categorical (0=no ROH, 1=ROH) |
| SNP (additive) | -0.054 | -0.152 | 0.043 | -1.089 | $2.76 \times 10^{-1}$ | continuous (hom(min)=0, het=1,hom(m aj)=2) |
| $F_{ROH}$ | -2.974 | -5.138 | -0.809 | -2.693 | $7.08 \times 10^{-3}$ | continuous (0-1) |
| Sex | 0.451 | 0.342 | 0.56 | 8.117 | $4.77 \times 10^{-16}$ | categorical (0=female, 1=male) |
| PC1 | -1.173 | -2.796 | 0.45 | -1.416 | $1.57 \times 10^{-1}$ | continuous |
| PC2 | 0.869 | -0.799 | 2.537 | 1.021 | $3.07 \times 10^{-1}$ | continuous |
| PC3 | 0.138 | -1.52 | 1.796 | 0.163 | $8.70 \times 10^{-1}$ | continuous |
| PC4 | -0.127 | -1.809 | 1.556 | -0.148 | $8.83 \times 10^{-1}$ | continuous |
| PC5 | -1.063 | -2.772 | 0.646 | -1.219 | $2.23 \times 10^{-1}$ | continuous |
| PC6 | -0.889 | -2.535 | 0.757 | -1.059 | $2.90 \times 10^{-1}$ | continuous |
| PC7 | -1.403 | -3.068 | 0.263 | -1.65 | $9.89 \times 10^{-2}$ | continuous |
| term | estimate (log-OR) | CI (2.5%) | CI (97.5%) | z-value | p-value | Info |
| PC8 | -2.846 | -4.525 | -1.168 | -3.323 | $8.90 \times 10^{-4}$ | continuous |
| PC9 | 1.617 | -0.044 | 3.278 | 1.909 | $5.63 \times 10^{-2}$ | continuous |
| PC10 | -0.373 | -2.113 | 1.366 | -0.421 | $6.74 \times 10^{-1}$ | continuous |

(continued)

| Random effect (standard deviation) | | | | | | |
|---|---|---|---|---|---|---|
| Birth year | 0.104 | NA | NA | NA | NA | n=9 |
| OLRE | 0.679 | NA | NA | NA | NA | n=940 |

**Table 18: Top 30 SNPs (0.0001%) in GWAS.**

| snp | estimate | p_val | state | chromosome | position | allele.1 | allele.2 |
|---|---|---|---|---|---|---|---|
| rs69045681 | -3.631 | 1.40E-07 | roh_0 | 16 | 8037334 | A | G |
| rs397353926 | -3.584 | 2.06E-07 | roh_0 | 16 | 8044639 | T | C |
| rs1143204553 | -5.211 | 5.23E-06 | roh_0 | 1 | 186974288 | C | T |
| rs1137456765 | -5.188 | 5.72E-06 | roh_0 | 1 | 186976104 | T | C |
| rs1139552972 | -5.189 | 5.75E-06 | roh_0 | 1 | 187062853 | T | C |
| rs1151439841 | -5.194 | 5.87E-06 | roh_0 | 1 | 187015387 | T | C |
| rs394928190 | -3.567 | 6.92E-06 | roh_0 | 16 | 7968367 | T | C |
| rs1139343255 | -5.084 | 8.75E-06 | roh_0 | 1 | 187017598 | T | G |
| rs1147331227 | -5.259 | 9.91E-06 | roh_0 | 1 | 186984661 | A | C |
| rs1145430855 | -5.015 | 1.16E-05 | roh_0 | 1 | 187036936 | T | C |
| rs396172417 | -5.183 | 1.16E-05 | roh_0 | 1 | 185644415 | C | T |
| rs1146505878 | -5.080 | 1.52E-05 | roh_0 | 1 | 185608549 | C | A |
| rs396752178 | -5.080 | 1.68E-05 | roh_0 | 1 | 185636491 | A | G |
| rs396382798 | -5.067 | 1.82E-05 | roh_0 | 1 | 185568271 | A | G |
| rs1147322906 | -5.049 | 1.85E-05 | roh_0 | 1 | 185566840 | A | G |
| rs1141079518 | -4.952 | 1.95E-05 | roh_0 | 1 | 185533217 | C | T |
| rs1141861033 | -5.041 | 2.03E-05 | roh_0 | 1 | 185470516 | T | C |
| rs396183792 | -5.025 | 2.04E-05 | roh_0 | 1 | 185635559 | T | C |
| rs1151886222 | -5.026 | 2.11E-05 | roh_0 | 1 | 186415766 | A | G |
| rs396219063 | -4.872 | 2.20E-05 | roh_0 | 1 | 187088819 | C | T |
| rs1144695068 | -5.014 | 2.22E-05 | roh_0 | 1 | 185870376 | A | G |
| rs395011693 | -5.017 | 2.23E-05 | roh_0 | 1 | 185642354 | T | C |
| rs394975574 | -5.007 | 2.29E-05 | roh_0 | 1 | 185580371 | G | A |
| rs1151706224 | -4.862 | 2.29E-05 | roh_0 | 1 | 187089504 | C | T |
| rs395014129 | -4.862 | 2.29E-05 | roh_0 | 1 | 187090136 | C | T |
| rs1147162779 | -5.018 | 2.31E-05 | roh_0 | 1 | 185484964 | T | C |
| chr1_185633214 | -5.001 | 2.36E-05 | roh_0 | 1 | 185633214 | A | G |
| rs394541988 | -4.889 | 2.38E-05 | roh_0 | 1 | 185679126 | T | C |
| rs68578737 | -4.881 | 2.41E-05 | roh_0 | 1 | 185663797 | T | C |
| rs1150637373 | -4.881 | 2.41E-05 | roh_0 | 1 | 185700418 | A | G |

**Table 19: Regions identified as selected among non-winners compared to elite stallions in a composite selections signals analysis.**

| Chr | cluster_snps _N | Cluster region | N_ Gene s_in | Genes_ up | Genes_do wn | Genes_ all | max_score_ in_clu ster |
|---|---|---|---|---|---|---|---|
| chr14 | 37 | 50.91-52.88 | 6 | | | | 2.850 |
| chr4 | 110 | 75.46-80.93 | 19 | | | | 2.795 |
| chr14 | 33 | 41.24-43.21 | 26 | | | | 2.773 |
| chr1 | 61 | 3.43-6.39 | 5 | | | | 2.694 |
| chr5 | 82 | 16.98-20.13 | 25 | | | | 2.550 |
| chr8 | 38 | 85.33-86.91 | 3 | | | | 2.226 |
| chr2 | 20 | 85.29-86.22 | 5 | | | | 2.010 |
| chr1 | 25 | 0.47-1.91 | 9 | | | | 1.992 |
| chr3 | 21 | 17.23-18.2 | 44 | | | | 1.972 |
| chr18 | 6 | 30.76-31 | 1 | | | | 1.911 |
| chr1 | 13 | 183.31-183.88 | 8 | | PYGL | PYGL | 1.897 |
| chr25 | 5 | 3.83-4.35 | 1 | | | | 1.864 |
| chr14 | 8 | 63.89-64.22 | 0 | EFNA5 | | EFNA5 | 1.851 |
| chr17 | 4 | 35.2-35.26 | 1 | | | | 1.847 |
| chr1 | 2 | 114.52-114.6 | 1 | | | | 1.844 |

**Example 8**

**SNPs of interest**

No SNP reached genome-wide significance in the GWAS. The GWAS uncovered three SNPs within long ROH that were suggestive of significance. The top three ranked SNPs were on ECA1 (rs68492263, $P$ = 6.03 × 10$^{-6}$), ECA3 (rs1142345000, $P$ = 8.05 × 10$^{-6}$), and ECA 16 (rs1136876668, P = 8.24 × 10$^{-6}$) (Figure 18, Table 20).

On ECA1, a long ROH overlapped the minor allele T at SNP rs68492263 at position 96704107 bp. This SNP was in the second ranked region for the GWAS using ROH of all lengths (1:96704107-97202669) where the top SNP was at position 96719990 bp. On ECA16, a long ROH overlapped the minor allele C at SNP rs1136876668 at position 61350408 bp. On ECA3 a long ROH overlapped the minor allele G at SNP rs1142345000 at position 110575172 bp.

**Table 20: Top 30 (0.0001%) ranked SNPs in the GWAS**

| snp | estimate | p_val | state | Chr. | position | allele.1 | allele.2 |
|---|---|---|---|---|---|---|---|
| **rs68492263** | -0.38424 | 6.03E-06 | roh_0 | 1 | 96704107 | T | C |
| **rs1136876668** | -0.61656 | 8.05E-06 | roh_0 | 16 | 61350408 | C | A |
| **rs1142345000** | -0.43597 | 8.24E-06 | roh_0 | 3 | 110575172 | G | T |
| **rs1144643078** | -0.61013 | 1.03E-05 | roh_0 | 16 | 61316728 | C | T |
| **rs394291305** | -0.63378 | 1.18E-05 | roh_0 | 16 | 61485318 | A | G |
| **rs1144152381** | -0.59441 | 1.26E-05 | roh_0 | 16 | 62361235 | G | T |
| **rs1138954915** | -0.59903 | 1.62E-05 | roh_0 | 16 | 61881224 | G | A |
| **rs1143627458** | -0.44259 | 1.68E-05 | roh_0 | 3 | 110626960 | T | C |
| **rs1144264641** | -0.35406 | 1.77E-05 | roh_0 | 15 | 37623615 | G | T |

(continued)

| snp | estimate | p_val | state | Chr. | position | allele.1 | allele.2 |
|---|---|---|---|---|---|---|---|
| **rs1139578484** | -0.58732 | 1.79E-05 | roh_0 | 16 | 62359700 | A | G |
| **rs1141586753** | -0.55774 | 2.09E-05 | roh_0 | 16 | 61579357 | A | G |
| **rs396933426** | -0.59315 | 2.17E-05 | roh_0 | 16 | 61888299 | G | A |
| **rs1149103864** | -0.55269 | 2.26E-05 | roh_0 | 16 | 61282867 | T | C |
| **rs1151161986** | -0.43345 | 2.32E-05 | roh_0 | 3 | 110580051 | C | T |
| **rs1148520935** | -0.59097 | 2.38E-05 | roh_0 | 16 | 61955520 | T | C |
| **rs1138851722** | -0.33661 | 2.57E-05 | roh_2 | 3 | 13600864 | T | C |
| **rs1140720421** | -0.29383 | 2.67E-05 | roh_2 | 18 | 68535533 | A | G |
| **rs1141842338** | -0.42788 | 2.72E-05 | roh_0 | 3 | 110384214 | T | C |
| **rs1149520510** | -0.60002 | 2.90E-05 | roh_0 | 16 | 61980497 | C | T |
| **rs1144608958** | -0.42611 | 2.96E-05 | roh_0 | 3 | 110905597 | C | T |
| **rs395438793** | -0.33118 | 3.15E-05 | roh_2 | 3 | 13451508 | G | A |
| **rs394055886** | -0.42045 | 3.15E-05 | roh_0 | 16 | 51763459 | C | T |
| **rs1137710709** | -0.42488 | 3.25E-05 | roh_0 | 3 | 110890960 | G | A |
| **rs1145156936** | -0.60993 | 3.26E-05 | roh_0 | 16 | 62173781 | C | T |
| **rs395900184** | -0.41858 | 3.31E-05 | roh_0 | 16 | 52114617 | T | G |
| **rs396437371** | -0.34066 | 3.39E-05 | roh_0 | 3 | 13940199 | C | T |
| **rs1143815205** | -0.4136 | 3.39E-05 | roh_0 | 3 | 110738405 | C | T |
| **rs1137740805** | -0.60816 | 3.56E-05 | roh_0 | 16 | 61851562 | A | C |
| **rs1143089369** | -0.42332 | 3.58E-05 | roh_0 | 16 | 51769224 | A | C |
| **rs1148874071** | -0.33391 | 3.65E-05 | roh_2 | 3 | 13668298 | A | G |

**Example 9**

**Regions of interest in European and Australian GWAS**

There were several peaks with more than two SNPs within 1 Mb of each other clustered among the top 0.001% ranked SNPs (*i.e.* 297 SNPs) that were considered regions of interest for further examination (Table 21). For example, the ECA1 rs68492263 SNP was among 15 SNPs that clustered within a 1.6 Mb region. The ECA16 rs1136876668 SNP was within a cluster of 40 SNPs within a 12.25 Mb region approximately centred on the SNP. The ECA3 rs1142345000 SNP was among 27 SNPs that clustered within a 2.24 Mb region approximately centred on the SNP. Four other regions of interest on ECA3, 15, 16 and 18 contained more than 15 of the top SNPs and the highest ranked SNP within each of the regions was among the top 0.0001% (*i.e.* 30) SNPs.

**Table 21.** Regions of interest among top ranked 0.001% of GWAS SNPs with highest ranked SNP among top 0.0001% SNPs in long ROH (>5 Mb)

| Region of interest | | | | | | Top ranked SNP in region | | | | | | | | |
|---|---|---|---|---|---|---|---|---|---|---|---|---|---|---|
| Chr. | start bp | end bp | length Mb | No protein coding genes in 5Mb | no SNPs in top 100 SNPs | snp | estimate | p_val | state | Chr. | position | allele.1 | allele.2 | SNP rank |
| 1 | 96626510 | 98284133 | 1.66 | 30 | 15 | rs68492263 | -0.384 | 6.03E-06 | roh_0 | 1 | 96704107 | T | C | 1 |
| 16 | 54707379 | 66954937 | 12.25 | 15 | 40 | rs1136876668 | -0.617 | 8.05E-06 | roh_0 | 16 | 61350408 | C | A | 2 |
| 3 | 109434554 | 111674968 | 2.24 | 15 | 27 | rs1142345000 | -0.436 | 8.24E-06 | roh_0 | 3 | 110575172 | G | T | 3 |
| 15 | 34836277 | 37696834 | 2.86 | 20 | 31 | rs1144264641 | -0.354 | 1.77E-05 | roh_0 | 15 | 37623615 | G | T | 9 |
| 3 | 12330293 | 14427200 | 2.10 | 8 | 47 | rs1138851722 | -0.337 | 2.57E-05 | roh_2 | 3 | 13600864 | T | C | 16 |
| 18 | 68395895 | 69051761 | 0.66 | 22 | 15 | rs1140720421 | -0.294 | 2.67E-05 | roh_2 | 18 | 68535533 | A | G | 17 |
| 16 | 50903024 | 53259316 | 2.36 | 11 | 24 | rs394055886 | -0.420 | 3.15E-05 | roh_0 | 16 | 51763459 | C | T | 22 |

## Example 10

### Candidate genes associated with OMIM Disease in regions of interest

The BIOMART feature in the Ensembl horse genome database was used to search for candidate protein coding genes within 2.5 Mb up and downstream of the top ranked SNP in each of the six highest ranked regions *(i.e.* 5 Mb search region). The online DAVID functional annotation tool (Huang D.W., Sherman B.T., Tan Q., Collins J.R., Alvord W.G., Roayaei J., Stephens R., Baseler M.W., Lane H.C. & Lempicki R.A. (2007) The DAVID Gene Functional Classification Tool: a novel biological module-centric algorithm to functionally analyze large gene lists. Genome Biol 8, R183) was used to examine gene ontology (GO) terms and OMIM (Online Mendelian Inheritance in Man) diseases associated with each gene. Genes with associated OMIM diseases that have phenotypes that may affect exercise and racing were first considered as possible candidate genes responsible for the GWAS signals. A subset of these that are associated with limb deformities is shown in Table 22.

The ECA1 rs68492263 region contained 30 protein coding genes, seven of which were associated with OMIM disease. *ACAN* (aggrecan, 1:95255522-95320938) and *FANCI* (FA complementation group I, 1: 94927176-95017933) also had disease entries in OMIA (Online Mendelian Inheritance in Animals). *ACAN* is responsible for height variation in horse breeds and is a cause of severe limb deformity and dwarfism especially in miniature breeds. *FANCI* causes brachyspina in *Bos taurus* cattle breeds (OMIA 000151-9913); calves born with brachyspina can have severely reduced body weight, a shortened spine, long and thin limbs, a deformed lower jaw and a visible hump between the shoulder blades.

The ECA16 rs1136876668 region contained 15 protein coding genes, six of which were associated with OMIM disease. *TOP2B* (DNA topoisomerase II beta, 16:58927472-59028172) causes B-cell immunodeficiency, distal limb anomalies, and urogenital malformations. *SGO1* (shugoshin 1, 16:63604231-63615086) causes chronic atrial and intestinal dysrhythmia.

The ECA3 rs1142345000 region contained 15 protein coding genes, five of which were associated with OMIM disease. *TAPT1* (transmembrane anterior posterior transformation 1, 3:108963273-109023472) causes osteochondrodysplasia, in which the growth and development of bone, cartilage, and/or connective tissue is impaired and usually appears as dwarfism. *NKX3-2* (NK3 homeobox 2, 3:111307951-111311578) causes Spondylo-megaepiphyseal-metaphyseal dysplasia; metaphyseal dysplasia and epiphyseal dysplasia are malformations of the ends of long bones in the arms and legs.

The ECA15 rs1144264641 region contained 20 protein coding genes, five of which were associated with OMIM disease. *SLC1A4* (solute carrier family 1 member 4, 15: 37746505-37769073) causes Spastic tetraplegia, an autosomal recessive neurodevelopmental disorder characterized by severely impaired global development in early infancy. Most patients are unable to achieve independent walking or speech; some patients have seizures. *WDPCP* (WD repeat containing planar cell polarity effector, 15:38995867-39351568) regulates cellular proliferation in the developing limb and causes congenital heart defects, hamartomas of tongue, and polysyndactyly.

The ECA3 rs1138851722 region contained 8 protein coding genes, two of which were associated with OMIM disease. *CNOT1* (CCR4-NOT transcription complex subunit 1, 3: 11211692-11324350) causes Vissers-Bodmer syndrome, which is characterized by global developmental delay with variably impaired intellectual development, speech delay, motor delay, and behavioural abnormalities apparent from infancy. Other common features include growth impairment abnormalities, hypotonia, and distal skeletal defects, such as foot and hand deformities.

The ECA18 rs1140720421 SNP at position 68535533 bp was within 2.5 Mb of *MSTN,* the gene selected for sprint racing (muscle hypertrophy) in thoroughbreds, but did not overlap the neighbouring region on ECA18 that is associated with fracture risk in thoroughbreds.

The ECA2 rs395687529 SNP at position 39102139 bp was 825kb from *PLOD1,* the gene that causes fragile foal syndrome in warmblood horse breeds.

## Example 11

### Thoroughbred specific mutations in ACAN

Variants in the *ACAN* gene were identified in thoroughbred whole genome sequence that are not annotated in the horse Ensembl variant database (Table 23), including three missense variants and two in frame deletions.

## Example 12

### Candidate genes with predicted high impact variants

Next, the putative functional effects of mutations in the regions of interest were examined to identify candidate genes. Annotated protein-coding genes were considered as candidates if they contained *in silico* predicted high impact variants and had a GO function or evidence from the literature for functions that may be relevant to exercise or disease that may impact on racing.

Seven genes (*KIF7, FANCI, AGBL1, AKAP13, VPS54, OSBPL10*) had mutations predicted to have a high effect on the encoded protein and with functions that may be relevant to the athletic horse (Table 24).

**Table 22**: Candidate genes associated with OMIM Disease terms that result in limb deformities located within 5 Mb of the top SNP in the three highest ranked regions of interest. The number of variants identified characterised in thoroughbred whole genome sequence data that are predicted to have negative effects on the resulting protein are shown.

| Gene name | Gene description | chr | Gene start (bp) | Gene end (bp) | OMIM_DISEASE | Summary of OMIM disease phenotypes relevant to equine musculoskeletal conformation | chr | top SNP position | distance from top GWAS SNP (Mb) | Protein-coding gene variants in WGS |
|---|---|---|---|---|---|---|---|---|---|---|

| NKX3-2 | TAPT1 | TOP2B | ACAN |
|---|---|---|---|
| NK3 homeobox 2 | transmembrane anterior posterior transformation 1 | DNA topoisomerase II beta | Aggrecan |
| 3 | 3 | 16 | 1 |
| 111307951 | 108963273 | 58927472 | 95255522 |
| 111311578 | 109023472 | 59028172 | 95320938 |
| Spondylo-megaepiphyseal-metaphyseal dysplasia, | Osteochondrodysplasia, complex lethal, Symoens-Barnes-Gistelinck type, | B-cell immunodeficiency, distal limb anomalies, and urogenital malformations, | Short stature and advanced bone age, with or without early-onset osteoarthritis and/or osteochondritis dissecans, Spondyloepiphyseal dysplasia, Kimberley type, Spondyloepimetaphyseal dysplasia, aggrecan type, |
| Malformations of the ends of long bones in the arms and legs | Impaired growth and development of bone, cartilage, and/or connective tissue | Distal limb anomalies | Severe limb defects |
| 3 | 3 | 16 | 1 |
| 110575172 | 110575172 | 61350408 | 96704107 |
| 0.74 | 1.55 | 2.32 | 1.38 |
| | | | Missense variant: 18 Inframe deletion: 2 |

**Table 23:** Thoroughbred variants with predicted moderate effects on ACAN protein. The dwarfism mutation (95271115 bp) is italicized. Variants identified in Thoroughbred WGS but not catalogued in horse Ensembl variant database are highlighted in bold font.

| bp position | allele 1 | allele 2 | Consequence type | AA | AA coord | SIFT |
|---|---|---|---|---|---|---|
| 95270484 | T | C | missense variant | R/G | 2366 | 0.58 |

(continued)

| bp position | allele 1 | allele 2 | Consequence type | AA | AA coord | SIFT |
|---|---|---|---|---|---|---|
| 95271115 | T | A | missense variant | L/F | 2155 | 0.12 |
| 95271452 | G | A | missense variant | A/V | 2043 | 0.59 |
| 95272282 | T | C | missense variant | I/M | 1766 | 0.32 |
| 95272998 | C | G | missense variant | A/P | 1528 | 0.24 |
| **95273021** | **C** | **T** | **missense variant - new** | ENSECAT00000040213.1:C.4559G>A\|ENS ECAP00000032890.1:P.GLY1520GLU\| | | |
| **95273215** | **62bp DEL** | **G** | **inframe deletion - new** | ENSECAT00000040213.1:C.4302-4364DEL \|ENSECAP00000032890.1:P.PRO1442_GL U1462DEL | | |
| 95273323 | T | G | missense variant | L/F | 1419 | 0.58 |
| **95273484** | **G** | **T** | **missense variant - new** | ENSECAT00000040213.1:C.4096C>A\|ENS ECAP00000032890.1:P.LEU1366ILE | | |
| 95274059 | G | A | missense variant | A/V | 1174 | 1 |
| 95274276 | C | T | missense variant | V/I | 1102 | 0.24 |
| 95274281 | T | C | missense variant | E/G | 1100 | 0.24 |
| **95274294** | **A** | **G** | **missense variant - new** | NSECAT00000040213.1:C.3286T>C\|ENSE CAP00000032890.1:P.PHE1096LEU | | |
| **95274300** | **T** | **C** | **missense variant - new** | ENSECA T00000040213.1:C.3280A>G\|ENS ECAP00000032890.1:P.SER1094GLY | | |
| 95274303 | T | G | missense variant | I/L | 1093 | 0.5 |
| **95274430** | **62bp DEL** | **A** | **inframe deletion - new** | ENSECAT00000040213.1:C.3087_3149DEL \|ENSECAP00000032890.1:P.LEU1030_ASP 1050DEL | | |
| 95274878 | G | A | missense variant | A/V | 901 | 1 |
| 95284467 | C | T | missense variant | V/I | 445 | 0.4 |
| 95284551 | G | C | missense variant | P/A | 417 | 0.81 |
| 95293801 | C | A | missense variant | V/L | 9 | 0.01 |

**Table 24:** Candidate genes with in silico predicted high impact variants in top ranked regions of interest

| chr | top SNP region | Gene | high impact variants | GO terms / OMIM_ DISEASE | Associated phenotypes |
|---|---|---|---|---|---|
| 1 | 96704107 | KIF7 | FRAMESHIFT_V ARIANT: 1 | Acrocallosal syndrome, Joube rt syndrome 12, Al-Gazali-Bakalinova syndrome, Hydro lethal us syndrome 2, | Idiopathic scoliosis Expressed throughout limb development, deletion has distinct effects on limb formation - ranging from severe truncation to polydactyly. Causes acrocallosal syndrome with polydactyly, brain abnormalities and cleft palate |
| | | FANCI | START_LOST | Fanconi anemia, complementation group I, | Brachyspina in cattle |
| | | AGBL1 | SPLICE_DONO R_VARIANT: 2 | Corneal dystrophy, Fuchs endothelial, 8, | Reproductive seasonality in sheep |
| | | AKAP13 | STOP_GAINED &FRAMESHIFT_ VARIANT: 2 | Heart development, bone development, cardiac muscle cell differentiation, cell growth involved in cardiac muscle cell development, energic receptor signaling pathway, adrenergic receptor signaling pathway involved in heart process, | Compulsive behaviours. Deficiency in early osteogenesis with a corresponding reduction in osteoblast number. Regulates aspects of $\beta$-adrenergic-induced cardiac hypertrophy. |
| 16 | 54707379 | OSBPL10 | START_LOST: 1, STOP_LOST: 1 | lipid transport | Pig birth weight |
| 15 | 37623615 | VPS54 | FRAMESHIFT_V ARIANT: 2 | regulation of growth, musculo skeletal movement | Wobbler mouse |

Inbreeding in livestock populations is difficult to avoid due to the often closed nature of the gene pool. Nonetheless, limiting inbreeding is likely to reduce the impact of undesirable inherited features. Until now, the functional or clinical consequences of inbreeding in the thoroughbred population were not known. Here it is shown, using SNP genotypes for a large cohort of thoroughbred horses in two of the major global breeding regions, that inbreeding depression is a genome-wide phenomenon significantly impacting on the viability of a horse to ever race. Given the current pedigree structure of thoroughbreds, avoiding breeding close relatives is challenging and the increasing levels of inbreeding in the population indicates that existing strategies to mitigate inbreeding may be inadequate. Some breeders consider that the duplication of influential ancestral horses in a pedigree may be advantageous and there are numerous examples of close pedigree inbreeding that result in successful racehorses. Indeed, here it is shown that inbreeding in the distant pedigree, measured as $F_{ROH\_short}$, is not disadvantageous to the breeding goal. However, more recently shared common ancestors, indicated by $F_{ROH\_long}$, have a considerable negative impact on the viability of a horse for racing and contribute to wastage in the population. Although not quantified here, it is likely that these long ROH contain a higher proportion of rare, deleterious alleles, which cumulatively cause the inbreeding depression observed.

Large-effect mutations are expected to be more easily purged by selection. However, here as well as describing the genome-wide architecture of inbreeding, a relatively common (10.1%) haplotype with an auxiliary effect on probability to race was identified. This haplotype did not overlap any known locus under selection for thoroughbreds. Nonetheless, there was a higher than expected frequency of the THR14 haplotype among stallions, which occurs in one in six breeding stallions in this sample. Estimating from the frequency of the haplotype in this sample (10.1%), among the 8,542 foals registered in Ireland in 2020, 87 are expected to be homozygous for the THR14 haplotype with 1,551 carriers.

*EFNA5* (ECA14: 63,365,481-63,631,761 bp), which encodes the ephrin ligand, ephrin-A5, is a compelling candidate gene for the negative effect of the THR14 haplotype on racing. Ephrin A5 is a member of the ephrin family of ligands and receptors that are broadly expressed during tissue development and repair. Ephrin A5 is involved in neonatal muscle development and regeneration, regulates cardiomyocytes, and is associated with growth traits and digital cushion in cattle. Ephrin ligand-receptor signalling also has a well characterised role in skeletal development and bone function. Generally, ephrin A member ligands bind to ephrin A receptors, however ephrin A5 is known to bind to the ephrin B2 receptor, which is encoded by a gene involved in osteogenic control and hip fracture in humans. The related ephrin B1 protein is required for the fracture repair process. Ephrin A5 has been proposed as a negative regulator of osteogenic differentiation and is highly expressed in cartilage where it participates in tissue growth and regeneration. Although articular cartilage is a highly resilient tissue, it has a poor capacity for regeneration and articular cartilage damage in horses is one of the most common causes of catastrophic musculoskeletal injury. Pre-existing degenerative articular cartilage and bone lesions have been commonly identified in euthanised horses post-injury resulting from cumulative damage to the tissues from repetitive strain during exercise.

In this regard, the THR14 haplotype overlaps a large suggestive GWAS peak on ECA14 (14: 55530859-70816518) for osteochondrosis in the Belgian Warmblood breed. Osteochondrosis is one of the most common skeletal diseases in the horse affecting cartilage and bone, causing inflammation of joints, pain, lameness and a decrease in athletic performance.

There are many reasons that a foal may not become a viable racehorse. However, musculoskeletal fractures are the most common cause of death at all stages of development, training, and racing and are a major concern for racehorse welfare. The known biological functions of *EFNA5* and the haplotype association with probability of racing that we report here, leads to the hypothesis that it may play a role in musculoskeletal injury risk, however this must be tested in a population of horses with well-defined phenotypes. While the nature of the hypothesised causative mutation on THR14 is not known, the present invention provides strong evidence for the presence of a mutation with a negative effect on racing located within this haplotype that has not previously been described.

In summary, the introduction of genome-enabled breeding strategies to avoid the production of long homozygous stretches in the genome and homozygosity of the THR14 haplotype could improve economic returns for breeders and positively impact animal welfare. Therefore, industry-guided monitoring of genome-wide inbreeding over time will be important to maintain sustainable populations of horses, with particular attention focused on higher resolution information that can be obtained for deleterious haplotypes such as THR14.

For Thoroughbreds bred and raced in Europe and Australia/New Zealand long ROH (>5 Mb) in the genome have a strong negative effect on the probability of a horse ever racing, with no effect from short ROH, indicating that recent inbreeding events are the cause of inbreeding depression for the racing trait in these populations. In a genome-wide association study (GWAS) for ROH effects on probability of ever racing we previously identified a single large effect locus (THR14) on ECA14 when ROH of all lengths were used (Figure 1). The THR14 haplotype contains the *EFNA5* gene that functions in cartilage growth and regeneration, and bone remodelling, and it has been hypothesised that a mutation in this haplotype may contribute to catastrophic musculoskeletal injury risk. Horses homozygous for the THR14 haplotype are three times less likely to ever race than other horses.

Since long ROH, in particular, are responsible for the inbreeding depression associated with the racing trait, here we performed a ROH-based GWAS for the effects of long ROH (ROH >5 Mb only) to detect large effect loci involved in inbreeding depression arising from recent inbreeding events. We examined ROH in >6,000 thoroughbred horses originating from Europe and Australasia, of which -13% never raced. The aim was to further characterise genes that may be underpinning traits that contribute to the large number of thoroughbred foals born that never race, which has been estimated between 35-50%. The identification of harmful haplotypes will allow for more precise selective breeding of horses to reduce the occurrence of inherited conditions in foals that impact on economic returns for breeders and animal welfare.

In the present study, in a ROH (>5Mb) GWAS, haplotypes that may contain genes that impact on the viability of a horse for racing have been identified. The goal of thoroughbred breeding is to produce viable foals that remain healthy and sound in order to realise their value by winning races and returning for breeding. Despite the breeding goal, a very large proportion (35-55%) of thoroughbred foals born never race. There are numerous reasons that a thoroughbred horse may fail to race, however musculoskeletal injuries are a major contributor to wastage and are the most common cause of death at all stages of development, training and racing. For foals, congenital defects are the most common (20%) cause of euthanasia, and of these musculoskeletal conformational defects (15%), with flexural deformities that result in contracture of the joints of the forelimbs the most prevalent. The requirement for euthanasia likely represents the severity of the deformity, unlikely to respond to surgical or other interventions. Developmental orthopaedic disease (DOD) can be surgically treated but may impact on sales price and on racing performance. As many as 11.3% of foals are treated for abnormal limb development, with angular limb deformities (ALDs) and physeal dysplagia the conditions most commonly treated. The prognosis for horses treated early for ALDs is generally good, and many horses go on to race and breed. Therefore, where genetic risk markers may be present in the population, breeding of horses with genetic defects is likely to propagate the issue.

Inbreeding, the mating of related animals, increases the chance for recessive mutations to be exposed and result in an aberrant phenotype. This study investigates the effects of SNPs in long ROH, generated from recent inbreeding events, on the failure of horses to race. There was no further information for the reasons the horses did not race and therefore no a *priori* consideration for specific functional ontologies or disease categories for the selection of candidate genes, other than identifying functions and/or phenotypes that may affect racing.

This approach did not aim to identify lethal haplotypes or mutations that may cause embryonic death, early abortion or neonatal death, since the majority of horses included in the study were sampled at -20 months old. The analytical approach differs from a genome scan designed to identify the absence or depletion of haplotypes that may indicate lethality.

Five candidate genes were identified in the top three regions of interest that in humans cause severe limb defects (*ACAN, KIF7, NKX3-2, TAPT1, TOP2B*).

The ECA1 region defined by the rs68492263SNP contained three genes of interest *(ACAN, KIF7* and *FANCI)*. *KIF7* is expressed during limb development and deletion of the gene results in limb defects. In the thoroughbred WGS data, a frameshift mutation (predicted high impact) was identified in KIF7 (Table 24). Mutations in *FANCI* cause brachyspina in cattle. In the thoroughbred WGS data, a start lost mutation (predicted high impact) was identified in FANCI (Table 24).

In *ACAN,* thoroughbred-specific mutations not previously reported in other horse breeds were identified including four new missense variants and two in frame deletions. In human populations and other animal species *ACAN* mutations result in short stature. For example, in cattle mutations are associated with dwarfism in the Dexter breed, with animals displaying abnormal cartilage development, and lethal chondrodyspasia in miniature Zebu. In horses, *ACAN is* one of several genes associated with height variation. In miniature horses and miniature Shetland ponies, *ACAN* mutations result in chondrodysplastic dwarfism, distinguished from short stature by evidence of skeletal dysplasia. The phenotypic consequences of different mutations in the gene are variable within and between species including short stature, minor skeletal defects including brachydactyly, and spondyloepiphyseal dysplasia and chondrodysplasia, and therefore a simple genotype to phenotype correlation cannot be clearly defined. Nonetheless, it is known that the encoded protein, aggrecan, is critical to the function of the cartilage growth plate and in miniature horses expression of the gene is significantly higher in articular cartilage from affected horses compared to normal animals. None of the variants identified for dwarfism phenotypes in other horse breeds (g.95291270del (rs1095048841), g.95284530C>T (ERP107353), g.95282140C>G (rs1095048823) and g.95257480_95257500del (rs1095048839) designated as D1, D2, D3* and D4 respectively) were identified in the thoroughbred WGS. The existence of additional *ACAN* alleles causing dwarfism in other breeds has been proposed since the dwarfism phenotype was observed in animals that were heterozygotes for the described mutations. Furthermore, genotypes for the different mutations have been observed to result in variable phenotypes. These results suggest a range of mutations in *ACAN* contribute to variable limb deformities in horses. It is proposed that the mutations that we have identified in the thoroughbred may contribute to limb deformities that are commonly observed in neonatal thoroughbred foals.

The ECA16 region defined by the rs1136876668 SNP contained two genes of interest (*TOP2B* and *UBE2E1)*. *TOP2B* encodes the DNA topoisomerase II beta protein, associated with OMIM disease BILU syndrome, which can present with mild limb deformities (OMIM # 609296). *UBE2E1* encodes ubiquitin conjugating enzyme E2 E1, which is expressed in skeletal muscle and protects against muscle atrophy.

Two candidate genes were identified on ECA3 defined by the rs1142345000 SNP. *TAPT1* encodes the transmembrane anterior posterior transformation 1 protein which causes Osteochondrodysplasia, complex lethal, Symoens-Barnes-Gistelinck type. *NKX3-2* encodes NK3 homeobox 2 which causes Spondylo-megaepiphyseal-metaphyseal dysplasia, a rare disease in humans in which limbs are relatively long and flexion contractures of the distal joints have been observed. Considering the very high prevalence of angular limb deformities and joint contractures in foals, it is highly likely that one or more of these genes contribute to the phenotypes associated with inherited conformational disorders in the thoroughbred. No high or moderate effect mutations were identified in *TAPT1* or *NKX3-2*. However, using the thoroughbred WGS data, a focus was on the identification of protein-coding variants, which precludes the identification of structural variants such as large insertions/deletions and chromosomal rearrangements, or sequence variants in genomic regulatory elements that also contribute to the gene regulatory networks underlying complex traits. Given the high linkage disequilibrium observed in the thoroughbred genome, a causative mutation was expected to be present in the GWAS region, given the signal associated with the ECA3 SNP.

Validation of the association of the haplotypes with developmental phenotypes will be required to confirm their contribution to specific forms of limb deformities. However, given the observation of a considerable number of genes associated with developmental diseases in humans, the very high prevalence of limb deformities in foals and the association of the haplotypes with failure to race, these haplotypes may be developed into genetic tests to inform breeding decisions to limit the negative consequences of inbreeding.

Knowledge of the carrier status of the harmful haplotypes could result in economic returns for breeders, and improved animal welfare. These haplotypes should be monitored in the population to safeguard the future of the thoroughbred population and inform population-wide management strategies.

The identification of harmful haplotypes will allow for more precise selective breeding of horses to reduce the occurrence of inherited conditions in foals that impact on economic returns for breeders and animal welfare.

**Claims**

1. A method of determining the likelihood of a horse to race, the method comprising the steps of:

   (a) identifying at least one biomarker in a sample from the horse;
   (b) comparing the at least one biomarker with a respective reference biomarker;

   wherein deviation of the at least one biomarker from the respective reference biomarker is indicative that a horse is unlikely to race.

2. A method according to claim 1, wherein the method is a method of determining the athletic or physical performance or ability of a horse, wherein deviation of the at least one biomarker from the respective reference biomarker is indicative of poor or less than average athletic or physical performance or ability of the horse.

3. A method according to claim 1, wherein the method is a method of determining a trait associated with athletic or physical performance or ability of a horse, wherein deviation of the at least one biomarker from the respective reference biomarker is indicative of the presence of a trait associated with athletic or physical performance or ability of the horse.

4. A method according to claim 3, wherein the trait is an injury, optionally a musculoskeletal injury.

5. A method according to any one of claims 1-4, wherein the at least one biomarker is a mutation selected from an insertion, a deletion, a substitution, an in frame mutation, a splice site mutation, a frameshift mutation, a transition mutation, a transversion mutation, a synonymous substitution mutation, a nonsynonymous substitution mutation, a missense mutation, and a nonsense mutation.

6. A method according to any one of claims 1-5, wherein the at least one biomarker is located in a chromosome selected from ECA14, ECA1, ECA3, ECA15, ECA16, and ECA18.

7. A method according to claim 6, wherein the at least one biomarker is located at a position (bp) of the chromosome ECA14 from 63424953 to 64382244.

8. A method according to claim 6 or 7, wherein the nucleotide located at each position (bp) of the chromosome ECA14 selected from: 63424953, 63431653, 63474969, 63476246, 63479516, 63485221, 63490350, 63518338, 63538068, 63554928, 63562113, 63577470, 63599074, 63619660, 63628976, 63630959, 63645752, 63681303, 63682826, 63684190, 63688573, 63695607, 63701442, 63709333, 63717804, 63736883, 63747455, 63761512, 63766293, 63771208, 63772024, 63783376, 63802033, 63825182, 63848624, 63850407, 63883487, 63890801, 63892618, 63898547, 63899399, 63905325, 63909899, 63933713, 63972605, 63974722, 63975161, 63987126, 63987230, 64000803, 64001578, 64007753, 64010233, 64028113, 64065892, 64080217, 64090380, 64092331, 64133314, 64134277, 64143098, 64146817, 64161221, 64164323, 64168280, 64174000, 64183785, 64184542, 64188500, 64193570, 64201657, 64212192, 64213227, 64214043, 64218266, 64223584, 64224027, 64231842, 64239433, 64302645, 64302781, 64347307, 64358002, and 64382244 is a nucleic acid selected from a nucleic acid sequence selected from any one of: SEQ ID NOs 1-9.

9. A method according to claim 8, wherein the nucleotide located at each position (bp) of the chromosome ECA14 is the nucleotide located at the respective nucleic acid position of the nucleic acid sequence defined by SEQ ID NO 3.

10. A method according to claim 6 or 7, wherein the at least one biomarker is located at position 63883487bp of the chromosome ECA14.

11. A method according to any one of claims 1-5, wherein the at least one biomarker is located within or adjacent a gene selected from EFNA5, ACAN, FANCI, TOP2B, SGO1, TAPT1, NKX3-2, SLC1A4, WDPCP, CNOT1, MSTN, PLOD1, KIF7, FANCI, AGBL1, AKAP13, VPS54, OSBPL10; FER, FBXL17; eca-mir-885, and PYGL.

12. A method according to any one of claims 1-5, wherein the at least one biomarker is a single nucleotide polymorphism selected from: rs1144708552; rs68492263; rs1136876668, rs1144643078, rs394291305, rs1144152381, rs1138954915, rs1139578484, rs1141586753, rs396933426, rs1149103864, rs1148520935, rs1149520510, rs394055886, rs1145156936, rs395900184, rs1137740805, rs1143089369; rs1142345000, rs1143627458, rs1151161986, rs1138851722, rs1141842338, rs1144608958, rs395438793, rs1137710709, rs396437371, rs1143815205, rs1148874071; rs1144264641; rs1140720421; rs395101612, rs1138670132, rs1138492247, rs68958215, rs1149870931, rs396583407, rs1151800515, rs68958240, rs1136187498, rs395992427, rs1148244706, rs1135858353, rs1147391875, rs1143032686, rs394533891, rs1135859848, rs1151500701, rs396870088, rs394409489, rs394937599, rs396647223, rs68959721, rs68959726, rs1144890403, rs1138630473, rs68959750, rs1143223768, rs68990125, rs68990128, rs1150574504, rs68990132, rs68990137, rs1138569150, rs397243755, rs396356157, rs396601898, rs1141210208, rs1136571164, rs395858230, rs394608468, rs393840383, rs68991720, rs397331375, rs1140055494, rs1145733844, rs1137832255, rs1150222254, rs1136163000, rs1139936607, rs68991747, rs1141962106, rs1147376386, rs1152140905, rs1152121276, rs68991774, rs68991779, rs1143460900, rs1138384269, rs68993404, rs1146520189, rs68993418, rs68993429, rs1138987584, rs396327114, rs1150551836, rs1136050352, rs1148269508, rs394550789, rs1145346312, rs68993440, rs1149414368, rs1143253668, rs1137955421, rs1146002769, rs1151408384, rs1147224992, rs1148055403, rs1139156858, rs1143522750, rs1138751848, rs396915511, rs394467627 and rs397448356.

13. A method according to claim 10, wherein the at least one biomarker is a single nucleotide polymorphism having a reference SNP cluster ID 1144708552 (rs1144708552).

14. A method according to any one of claims 1-13, wherein the method is a method of breeding or mating a horse, wherein deviation of the at least one biomarker from the respective reference biomarker is indicative that a horse is unsuitable for breeding or mating with a second horse having the same at least one biomarker deviating from the respective reference biomarker. Further optionally, deviation of the at least one biomarker from the respective reference biomarker identifies a horse that is unsuitable for breeding or mating with a second horse having the same at least one biomarker deviating from the respective reference biomarker.

15. A method of determining the likelihood of a horse to race, the method comprising the steps of:

    (a) identifying at least one run of homozygosity;
    (b) determining the length of the at least one run of homozygosity;
    (c) determining the length of a genome; and
    (d) comparing the length of the at least one run of homozygosity to the length of the genome;

    wherein a ratio of the length of the at least one run of homozygosity compared to the length of the genome is indicative that a horse is likely to race.

Figure 1

Figure 2

Figure 3

Figure 7

Figure 4

Figure 6

Figure 5

Figure 8

Figure 9

## Predicted probabilities of raced

EP 4 286 534 A1

Figure 10

Figure 11

49

Figure 12

Figure 14

ROH at GWAS hit (Chr 16, pos 8037334 (8.04 Mb))

Figure 13

Figure 18

Figure 13

Figure 16

Figure 17

## EUROPEAN SEARCH REPORT

Application Number

EP 22 17 6585

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/262915 A1 (HILL EMMELINE [IE] ET AL) 27 October 2011 (2011-10-27) | 1-6,11, 14 | INV. C12Q1/6876 |
| Y | * claims; [0007]-[0021]; [0072]-[0076] * | 7-10,12, 13 | |
| | ----- | | |
| X | JULIA METZGER ET AL: "Runs of homozygosity reveal signatures of positive selection for reproduction traits in breed and non-breed horses", BMC GENOMICS, BIOMED CENTRAL LTD, LONDON, UK, vol. 16, no. 1, 9 October 2015 (2015-10-09), page 764, XP021229697, ISSN: 1471-2164, DOI: 10.1186/S12864-015-1977-3 * page 9, left-hand column; page 11, right-hand column * | 15 | |
| | ----- | | |
| X | US 2011/223600 A1 (HILL EMMELINE [IE] ET AL) 15 September 2011 (2011-09-15) * [0014]-[0050] * | 1-6,11, 14 | **TECHNICAL FIELDS SEARCHED (IPC)** |
| | ----- | | C12Q |
| Y | DR CLAUDIA DIERKS: "Molecular genetic analysis of quantitative trait loci (QTL) for osteochondrosis in Hanoverian warmblood horses", THESE DE DOCTORAT PRESENTÉE AU DÉPARTEMENT DE CHIMIE DE L'UNIVERSITÉ DE LAUSANNE POUR L'OBTENTION DU GRADE DE DOCTEUR ÈS SCIENCES,, 1 May 2006 (2006-05-01), page complete, XP002424022, * tables page 40; page 52; table 3, page 62 * | 7-10,12, 13 | |
| | ----- | | |
| | -/-- | | |

The present search report has been drawn up for all claims

| Place of search | Date of completion of the search | Examiner |
|---|---|---|
| Munich | 10 November 2022 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## EUROPEAN SEARCH REPORT

Application Number

EP 22 17 6585

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Hill Emmeline W ET AL: "Inbreeding depression and the probability of racing in the Thoroughbred horse", Proc. R. Soc. B, 29 June 2022 (2022-06-29), XP055974252, DOI: https://doi.org/10.1098/rspb.2022.0487 Retrieved from the Internet: URL:https://royalsocietypublishing.org/doi/pdf/10.1098/rspb.2022.0487 [retrieved on 2022-10-24] * the whole document * | | |

TECHNICAL FIELDS SEARCHED (IPC)

The present search report has been drawn up for all claims

| Place of search | Date of completion of the search | Examiner |
|---|---|---|
| Munich | 10 November 2022 | Hennard, Christophe |

## ANNEX TO THE EUROPEAN SEARCH REPORT
## ON EUROPEAN PATENT APPLICATION NO.

EP 22 17 6585

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2022

| Patent document cited in search report | Publication date | Patent family member(s) | Publication date |
|---|---|---|---|
| US 2011262915 A1 | 27-10-2011 | US 2011262915 A1 | 27-10-2011 |
| | | US 2014189894 A1 | 03-07-2014 |
| | | US 2016215335 A1 | 28-07-2016 |
| | | US 2020115751 A1 | 16-04-2020 |
| US 2011223600 A1 | 15-09-2011 | AU 2009290452 A1 | 18-03-2010 |
| | | EP 2352850 A1 | 10-08-2011 |
| | | JP 5667057 B2 | 12-02-2015 |
| | | JP 2012501671 A | 26-01-2012 |
| | | NZ 591711 A | 30-11-2012 |
| | | PT 2352850 E | 25-06-2014 |
| | | US 2011223600 A1 | 15-09-2011 |
| | | WO 2010029527 A1 | 18-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BROWNING BL ; ZHOU Y ; BROWNING SR.** A One-Penny Imputed Genome from Next-Generation Reference Panels. *Am J Hum Genet,* 2018, vol. 103 (3), 338-48
- **PURCELL S. ; NEALE B. ; TODD-BROWN K. ; THOMAS L. ; FERREIRA M.A. ; BENDER D. ; MALLER J. ; SKLAR P. ; DE BAKKER P.I. ; DALY M.J.** PLINK: a tool set for whole-genome association and population-based linkage analyses. *Am J Hum Genet,* 2007, vol. 81, 559-75
- **WADE CM ; GIULOTTO E ; SIGURDSSON S ; ZOLI M ; GNERRE S ; IMSLAND F et al.** Genome sequence, comparative analysis, and population genetics of the domestic horse. *Science,* 2009, vol. 326 (5954), 865-7
- **MCQUILLAN R ; LEUTENEGGER AL ; ABDEL-RAHMAN R ; FRANKLIN CS ; PERICIC M ; BARAC-LAUC L et al.** Runs of homozygosity in European populations. *Am J Hum Genet,* 2008, vol. 83 (3), 359-72
- **BATES DM ; MÄCHLER M ; BOLKER B ; WALKER S.** Fitting linear mixed-effects models using lme4. *Journal of Statistical Software,* 2015, 67
- **PRYCE JE ; HAILE-MARIAM M ; GODDARD ME ; HAYES BJ.** Identification of genomic regions associated with inbreeding depression in Holstein and Jersey dairy cattle. *Genet Sel Evol,* 2014, vol. 46, 71

- **STOFFEL MA ; JOHNSTON SE ; PILKINGTON JG ; PEMBERTON JM.** Mutation load decreases with haplotype age in wild Soay sheep. *Evol Lett.,* 2021, vol. 5 (3), 187-95
- **GAO X ; STARMER J ; MARTIN ER.** A multiple testing correction method for genetic association studies using correlated single nucleotide polymorphisms. *Genet Epidemiol,* 2008, vol. 32 (4), 361-9
- **NG P.C. ; HENIKOFF S.** SIFT: Predicting amino acid changes that affect protein function. *Nucleic Acids Res,* 2003, vol. 31, 3812-4
- **MCLAREN W. ; GIL L. ; HUNT S.E. ; RIAT H.S. ; RITCHIE G.R. ; THORMANN A. ; FLICEK P. ; CUNNINGHAM F.** The Ensembl Variant Effect Predictor. *Genome Biol,* 2016, vol. 17, 122
- **JALALI SEFID DASHTI M. ; GAMIELDIEN J.** A practical guide to filtering and prioritizing genetic variants. *Biotechniques,* 2017, vol. 62, 18-30
- **HUANG D.W. ; SHERMAN B.T. ; TAN Q. ; COLLINS J.R. ; ALVORD W.G. ; ROAYAEI J. ; STEPHENS R. ; BASELER M.W. ; LANE H.C. ; LEMPICKI R.A.** The DAVID Gene Functional Classification Tool: a novel biological module-centric algorithm to functionally analyze large gene lists. *Genome Biol,* 2007, vol. 8, R183